(19) Europäisches Patentamt
European Patent Office
Office européen des brevets

(11) EP 3 091 080 A1

(12) EUROPEAN PATENT APPLICATION

(43) Date of publication:
09.11.2016 Bulletin 2016/45

(21) Application number: 15166355.6

(22) Date of filing: 05.05.2015

(51) Int Cl.:
*C12P 13/04* (2006.01) *C12N 9/10* (2006.01)
*C12N 9/20* (2006.01) *C12N 9/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicant: **Evonik Degussa GmbH**
**45128 Essen (DE)**

(72) Inventors:
- **Grammann, Katrin**
  **45739 Oer-Erkenschwick (DE)**
- **Hennemann, Hans-Georg**
  **50181 Bedburg (DE)**
- **Decker, Nicole**
  **44803 Bochum (DE)**
- **Kirchner, Nicole**
  **45665 Recklinghausen (DE)**
- **Reinecke, Liv**
  **45134 Essen (DE)**

# (54) PROCESS FOR PRODUCING ACYL AMINO ACIDS EMPLOYING LIPASES

(57) There is provided a microbial cell for producing at least one acyl amino acid, wherein the cell is genetically modified to comprise:
- a first genetic mutation that increases the expression relative to a wild type cell of at least one lipase (EC 3.1.1) ($E_1$) capable of hydrolysing at least one glyceride to at least one fatty acid; and
- a second and a third genetic mutation that increases the expression relative to a wild type cell of:
(i) an amino acid-N-acyl-transferase (EC 2.3.2) ($E_2$), and
(ii) an acyl-CoA synthetase (EC 6.2.1.1) ($E_3$) respectively

that enables the cell to convert the fatty acid to at least one acyl amino acid and wherein the cell has a reduced fatty acid degradation capacity.

EP 3 091 080 A1

SEQUENCE LISTING

<110> Evonik Industries AG

<120> A METHOD FOR PRODUCING ACYL AMINO ACIDS

<130> 201500025

<160> 13

<170> PatentIn version 3.5

<210> 1
<211> 294
<212> PRT
<213> Homo sapiens

<400> 1

```
Met Leu Val Leu His Asn Ser Gln Lys Leu Gln Ile Leu Tyr Lys Ser
1               5                   10                  15

Leu Glu Lys Ser Ile Pro Glu Ser Ile Lys Val Tyr Gly Ala Ile Phe
            20                  25                  30

Asn Ile Lys Asp Lys Asn Pro Phe Asn Met Glu Val Leu Val Asp Ala
        35                  40                  45

Trp Pro Asp Tyr Gln Ile Val Ile Thr Arg Pro Gln Lys Gln Glu Met
    50                  55                  60

Lys Asp Asp Gln Asp His Tyr Thr Asn Thr Tyr His Ile Phe Thr Lys
65                  70                  75                  80

Ala Pro Asp Lys Leu Glu Glu Val Leu Ser Tyr Ser Asn Val Ile Ser
                85                  90                  95

Trp Glu Gln Thr Leu Gln Ile Gln Gly Cys Gln Glu Gly Leu Asp Glu
            100                 105                 110

Ala Ile Arg Lys Val Ala Thr Ser Lys Ser Val Gln Val Asp Tyr Met
        115                 120                 125

Lys Thr Ile Leu Phe Ile Pro Glu Leu Pro Lys Lys His Lys Thr Ser
    130                 135                 140

Ser Asn Asp Lys Met Glu Leu Phe Glu Val Asp Asp Asp Asn Lys Glu
145                 150                 155                 160

Gly Asn Phe Ser Asn Met Phe Leu Asp Ala Ser His Ala Gly Leu Val
                165                 170                 175
```

Asn Glu His Trp Ala Phe Gly Lys Asn Glu Arg Ser Leu Lys Tyr Ile
180 185 190

Glu Arg Cys Leu Gln Asp Phe Leu Gly Phe Gly Val Leu Gly Pro Glu
195 200 205

Gly Gln Leu Val Ser Trp Ile Val Met Glu Gln Ser Cys Glu Leu Arg
210 215 220

Met Gly Tyr Thr Val Pro Lys Tyr Arg His Gln Gly Asn Met Leu Gln
225 230 235 240

Ile Gly Tyr His Leu Glu Lys Tyr Leu Ser Gln Lys Glu Ile Pro Phe
245 250 255

Tyr Phe His Val Ala Asp Asn Asn Glu Lys Ser Leu Gln Ala Leu Asn
260 265 270

Asn Leu Gly Phe Lys Ile Cys Pro Cys Gly Trp His Gln Trp Lys Cys
275 280 285

Thr Pro Lys Lys Tyr Cys
290

<210> 2
<211> 561
<212> PRT
<213> Artificial Sequence

<220>
<223> FadD Acyl-CoA synthase

<400> 2

Met Lys Lys Val Trp Leu Asn Arg Tyr Pro Ala Asp Val Pro Thr Glu
1 5 10 15

Ile Asn Pro Asp Arg Tyr Gln Ser Leu Val Asp Met Phe Glu Gln Ser
20 25 30

Val Ala Arg Tyr Ala Asp Gln Pro Ala Phe Val Asn Met Gly Glu Val
35 40 45

Met Thr Phe Arg Lys Leu Glu Glu Arg Ser Arg Ala Phe Ala Ala Tyr
50 55 60

Leu Gln Gln Gly Leu Gly Leu Lys Lys Gly Asp Arg Val Ala Leu Met
65 70 75 80

Met Pro Asn Leu Leu Gln Tyr Pro Val Ala Leu Phe Gly Ile Leu Arg

85 90 95

Ala Gly Met Ile Val Val Asn Val Asn Pro Leu Tyr Thr Pro Arg Glu
100 105 110

Leu Glu His Gln Leu Asn Asp Ser Gly Ala Ser Ala Ile Val Ile Val
115 120 125

Ser Asn Phe Ala His Thr Leu Glu Lys Val Val Asp Lys Thr Ala Val
130 135 140

Gln His Val Ile Leu Thr Arg Met Gly Asp Gln Leu Ser Thr Ala Lys
145 150 155 160

Gly Thr Val Val Asn Phe Val Val Lys Tyr Ile Lys Arg Leu Val Pro
165 170 175

Lys Tyr His Leu Pro Asp Ala Ile Ser Phe Arg Ser Ala Leu His Asn
180 185 190

Gly Tyr Arg Met Gln Tyr Val Lys Pro Glu Leu Val Pro Glu Asp Leu
195 200 205

Ala Phe Leu Gln Tyr Thr Gly Gly Thr Thr Gly Val Ala Lys Gly Ala
210 215 220

Met Leu Thr His Arg Asn Met Leu Ala Asn Leu Glu Gln Val Asn Ala
225 230 235 240

Thr Tyr Gly Pro Leu Leu His Pro Gly Lys Glu Leu Val Val Thr Ala
245 250 255

Leu Pro Leu Tyr His Ile Phe Ala Leu Thr Ile Asn Cys Leu Leu Phe
260 265 270

Ile Glu Leu Gly Gly Gln Asn Leu Leu Ile Thr Asn Pro Arg Asp Ile
275 280 285

Pro Gly Leu Val Lys Glu Leu Ala Lys Tyr Pro Phe Thr Ala Ile Thr
290 295 300

Gly Val Asn Thr Leu Phe Asn Ala Leu Leu Asn Asn Lys Glu Phe Gln
305 310 315 320

Gln Leu Asp Phe Ser Ser Leu His Leu Ser Ala Gly Gly Gly Met Pro
325 330 335

```
Val Gln Gln Val Val Ala Glu Arg Trp Val Lys Leu Thr Gly Gln Tyr
            340                 345                 350

Leu Leu Glu Gly Tyr Gly Leu Thr Glu Cys Ala Pro Leu Val Ser Val
        355                 360                 365

Asn Pro Tyr Asp Ile Asp Tyr His Ser Gly Ser Ile Gly Leu Pro Val
    370                 375                 380

Pro Ser Thr Glu Ala Lys Leu Val Asp Asp Asp Asp Asn Glu Val Pro
385                 390                 395                 400

Pro Gly Gln Pro Gly Glu Leu Cys Val Lys Gly Pro Gln Val Met Leu
                405                 410                 415

Gly Tyr Trp Gln Arg Pro Asp Ala Thr Asp Glu Ile Ile Lys Asn Gly
            420                 425                 430

Trp Leu His Thr Gly Asp Ile Ala Val Met Asp Glu Glu Gly Phe Leu
        435                 440                 445

Arg Ile Val Asp Arg Lys Lys Asp Met Ile Leu Val Ser Gly Phe Asn
    450                 455                 460

Val Tyr Pro Asn Glu Ile Glu Asp Val Val Met Gln His Pro Gly Val
465                 470                 475                 480

Gln Glu Val Ala Ala Val Gly Val Pro Ser Gly Ser Ser Gly Glu Ala
                485                 490                 495

Val Lys Ile Phe Val Val Lys Lys Asp Pro Ser Leu Thr Glu Glu Ser
            500                 505                 510

Leu Val Thr Phe Cys Arg Arg Gln Leu Thr Gly Tyr Lys Val Pro Lys
        515                 520                 525

Leu Val Glu Phe Arg Asp Glu Leu Pro Lys Ser Asn Val Gly Lys Ile
    530                 535                 540

Leu Arg Arg Glu Leu Arg Asp Glu Ala Arg Gly Lys Val Asp Asn Lys
545                 550                 555                 560

Ala


<210>  3
<211>  906
<212>  DNA
```

<213> Artificial Sequence

<220>
<223> synUcTE (an acyl-CoA thioesterase) gene (codon-optimized)

<400> 3
atgactctag agtggaaacc gaaaccaaaa ctgcctcaac tgctggatga tcacttcggt 60

ctgcacggtc tggtgtttcg tcgtactttc gcaattcgtt cttatgaagt gggtccagat 120

cgttctacct ccatcctggc cgtcatgaac cacatgcagg aagccaccct gaatcacgcg 180

aaatctgttg gtatcctggg tgatggtttc ggcactactc tggaaatgtc taaacgtgac 240

ctgatgtggg tagtgcgtcg cacccacgta gcagtagagc gctaccctac ttggggtgac 300

actgtggaag tcgagtgttg gattggcgcg tccggtaaca atggtatgcg tcgcgatttt 360

ctggtccgtg actgtaaaac gggcgaaatc ctgacgcgtt gcacctccct gagcgttctg 420

atgaacaccc gcactcgtcg cctgtctacc atcccggacg aagtgcgcgg tgagatcggt 480

cctgctttca tcgataacgt ggcagttaaa gacgacgaaa tcaagaaact gcaaaaactg 540

aacgactcca ccgcggacta catccagggc ggtctgactc cgcgctggaa cgacctggat 600

gttaatcagc atgtgaacaa cctgaaatac gttgcttggg tcttcgagac tgtgccggac 660

agcattttcg aaagccatca catttcctct tttactctgg agtaccgtcg cgaatgtact 720

cgcgactccg ttctgcgcag cctgaccacc gtaagcggcg gttctagcga ggcaggtctg 780

gtctgcgacc atctgctgca actggaaggc ggctccgaag tcctgcgtgc gcgtacggag 840

tggcgtccaa agctgacgga ttctttccgc ggcatctccg taattccggc ggaacctcgt 900

gtttaa 906

<210> 4
<211> 290
<212> PRT
<213> Thermomyces lanuginosus

<400> 4

Met Arg Ser Ser Leu Val Leu Phe Phe Val Ser Ala Trp Thr Ala Leu
1 5 10 15

Ala Ser Pro Ile Arg Arg Glu Val Ser Gln Asp Leu Phe Asn Gln Phe
20 25 30

Asn Leu Phe Ala Gln Tyr Ser Ala Ala Ala Tyr Cys Gly Lys Asn Asn
35 40 45

Asp Ala Pro Ala Gly Thr Asn Ile Thr Cys Thr Gly Asn Ala Cys Pro
50 55 60

Glu Val Glu Lys Ala Asp Ala Thr Phe Leu Tyr Ser Phe Glu Asp Ser

```
65                  70                  75                  80

Gly Val Gly Asp Val Thr Gly Phe Leu Ala Leu Asp Asn Thr Asn Lys
                85                  90                  95

Leu Ile Val Leu Ser Phe Arg Gly Ser Arg Ser Ile Glu Asn Trp Ile
            100                 105                 110

Gly Asn Leu Asn Phe Asp Leu Lys Glu Ile Asn Asp Ile Cys Ser Gly
        115                 120                 125

Cys Arg Gly His Asp Gly Phe Thr Ser Ser Trp Arg Ser Val Ala Asp
    130                 135                 140

Thr Leu Arg Gln Lys Val Glu Asp Ala Val Arg Glu His Pro Asp Tyr
145                 150                 155                 160

Arg Val Val Phe Thr Gly His Ser Leu Gly Gly Ala Leu Ala Thr Val
                165                 170                 175

Ala Gly Ala Asp Leu Arg Gly Asn Gly Tyr Asp Ile Asp Val Phe Ser
            180                 185                 190

Tyr Gly Ala Pro Arg Val Gly Asn Arg Ala Phe Ala Glu Phe Leu Thr
        195                 200                 205

Val Gln Thr Gly Gly Thr Leu Tyr Arg Ile Thr His Thr Asn Asp Ile
    210                 215                 220

Val Pro Arg Leu Pro Pro Arg Glu Phe Gly Tyr Ser His Ser Ser Pro
225                 230                 235                 240

Glu Tyr Trp Ile Lys Ser Gly Thr Leu Val Val Thr Arg Asn Asp Ile
                245                 250                 255

Val Lys Ile Glu Gly Ile Asp Ala Thr Gly Gly Asn Asn Gln Pro Asn
            260                 265                 270

Ile Pro Asp Ile Pro Ala His Leu Trp Tyr Phe Gly Leu Ile Gly Thr
        275                 280                 285

Cys Leu
    290


<210>  5
<211>  230
<212>  PRT
<213>  Pseudomonas putida
```

<400> 5

```
Met Ser Phe Ser Asn Tyr Lys Val Ile Ala Met Pro Val Leu Val Ala
1               5                   10                  15

Asn Phe Val Leu Gly Ala Ala Thr Ala Trp Ala Asn Glu Asn Tyr Pro
            20                  25                  30

Ala Lys Ser Ala Gly Tyr Asn Gln Gly Asp Trp Val Ala Ser Phe Asn
        35                  40                  45

Phe Ser Lys Val Tyr Val Gly Glu Glu Leu Gly Asp Leu Asn Val Gly
    50                  55                  60

Gly Gly Ala Leu Pro Asn Ala Asp Val Ser Ile Gly Asn Asp Thr Thr
65                  70                  75                  80

Leu Thr Phe Asp Ile Ala Tyr Phe Val Ser Ser Asn Ile Ala Val Asp
                85                  90                  95

Phe Phe Val Gly Val Pro Ala Arg Ala Lys Phe Gln Gly Glu Lys Ser
            100                 105                 110

Ile Ser Ser Leu Gly Arg Val Ser Glu Val Asp Tyr Gly Pro Ala Ile
        115                 120                 125

Leu Ser Leu Gln Tyr His Tyr Asp Ser Phe Glu Arg Leu Tyr Pro Tyr
    130                 135                 140

Val Gly Val Gly Val Gly Arg Val Leu Phe Phe Asp Lys Thr Asp Gly
145                 150                 155                 160

Ala Leu Ser Ser Phe Asp Ile Lys Asp Lys Trp Ala Pro Ala Phe Gln
                165                 170                 175

Val Gly Leu Arg Tyr Asp Leu Gly Asn Ser Trp Met Leu Asn Ser Asp
            180                 185                 190

Val Arg Tyr Ile Pro Phe Lys Thr Asp Val Thr Gly Thr Leu Gly Pro
        195                 200                 205

Val Pro Val Ser Thr Lys Ile Glu Val Asp Pro Phe Ile Leu Ser Leu
    210                 215                 220

Gly Ala Ser Tyr Val Phe
225                 230
```

<210> 6
<211> 448
<212> PRT
<213> Artificial Sequence

<220>
<223> FadL

<400> 6

```
Met Val Met Ser Gln Lys Thr Leu Phe Thr Lys Ser Ala Leu Ala Val
1               5                   10                  15

Ala Val Ala Leu Ile Ser Thr Gln Ala Trp Ser Ala Gly Phe Gln Leu
            20                  25                  30

Asn Glu Phe Ser Ser Ser Gly Leu Gly Arg Ala Tyr Ser Gly Glu Gly
        35                  40                  45

Ala Ile Ala Asp Asp Ala Gly Asn Val Ser Arg Asn Pro Ala Leu Ile
    50                  55                  60

Thr Met Phe Asp Arg Pro Thr Phe Ser Ala Gly Ala Val Tyr Ile Asp
65                  70                  75                  80

Pro Asp Val Asn Ile Ser Gly Thr Ser Pro Ser Gly Arg Ser Leu Lys
                85                  90                  95

Ala Asp Asn Ile Ala Pro Thr Ala Trp Val Pro Asn Met His Phe Val
            100                 105                 110

Ala Pro Ile Asn Asp Gln Phe Gly Trp Gly Ala Ser Ile Thr Ser Asn
        115                 120                 125

Tyr Gly Leu Ala Thr Glu Phe Asn Asp Thr Tyr Ala Gly Gly Ser Val
    130                 135                 140

Gly Gly Thr Thr Asp Leu Glu Thr Met Asn Leu Asn Leu Ser Gly Ala
145                 150                 155                 160

Tyr Arg Leu Asn Asn Ala Trp Ser Phe Gly Leu Gly Phe Asn Ala Val
                165                 170                 175

Tyr Ala Arg Ala Lys Ile Glu Arg Phe Ala Gly Asp Leu Gly Gln Leu
            180                 185                 190

Val Ala Gly Gln Ile Met Gln Ser Pro Ala Gly Gln Thr Gln Gln Gly
        195                 200                 205

Gln Ala Leu Ala Ala Thr Ala Asn Gly Ile Asp Ser Asn Thr Lys Ile
```

```
    210                 215                 220

Ala His Leu Asn Gly Asn Gln Trp Gly Phe Gly Trp Asn Ala Gly Ile
225                 230                 235                 240

Leu Tyr Glu Leu Asp Lys Asn Asn Arg Tyr Ala Leu Thr Tyr Arg Ser
                245                 250                 255

Glu Val Lys Ile Asp Phe Lys Gly Asn Tyr Ser Ser Asp Leu Asn Arg
            260                 265                 270

Ala Phe Asn Asn Tyr Gly Leu Pro Ile Pro Thr Ala Thr Gly Gly Ala
        275                 280                 285

Thr Gln Ser Gly Tyr Leu Thr Leu Asn Leu Pro Glu Met Trp Glu Val
    290                 295                 300

Ser Gly Tyr Asn Arg Val Asp Pro Gln Trp Ala Ile His Tyr Ser Leu
305                 310                 315                 320

Ala Tyr Thr Ser Trp Ser Gln Phe Gln Gln Leu Lys Ala Thr Ser Thr
                325                 330                 335

Ser Gly Asp Thr Leu Phe Gln Lys His Glu Gly Phe Lys Asp Ala Tyr
            340                 345                 350

Arg Ile Ala Leu Gly Thr Thr Tyr Tyr Tyr Asp Asp Asn Trp Thr Phe
        355                 360                 365

Arg Thr Gly Ile Ala Phe Asp Asp Ser Pro Val Pro Ala Gln Asn Arg
    370                 375                 380

Ser Ile Ser Ile Pro Asp Gln Asp Arg Phe Trp Leu Ser Ala Gly Thr
385                 390                 395                 400

Thr Tyr Ala Phe Asn Lys Asp Ala Ser Val Asp Val Gly Val Ser Tyr
                405                 410                 415

Met His Gly Gln Ser Val Lys Ile Asn Glu Gly Pro Tyr Gln Phe Glu
            420                 425                 430

Ser Glu Gly Lys Ala Trp Leu Phe Gly Thr Asn Phe Asn Tyr Ala Phe
        435                 440                 445

<210>  7
<211>  45
<212>  DNA
<213>  Artificial Sequence
```

<220>
<223> ompAfwd

<400> 7
cggataacaa tttcacacag aggagaaatt aactatgaaa aaaac 45

<210> 8
<211> 40
<212> DNA
<213> Artificial Sequence

<220>
<223> LipT1-rev

<400> 8
gggtaccgag ctcgcatgcg tcacaggcag gtgccaatca 40

<210> 9
<211> 4541
<212> DNA
<213> Artificial Sequence

<220>
<223> vector pQE80L-kan

<400> 9
ctcgagaaat cataaaaaat ttatttgctt tgtgagcgga taacaattat aatagattca 60

attgtgagcg gataacaatt tcacacagaa ttcattaaag aggagaaatt aactatgaga 120

ggatcgcatc accatcacca tcacggatcc gcatgcgagc tcggtacccc gggtcgacct 180

gcagccaagc ttaattagct gagcttggac tcctgttgat agatccagta atgacctcag 240

aactccatct ggatttgttc agaacgctcg gttgccgccg ggcgtttttt attggtgaga 300

atccaagcta gcttggcgag attttcagga gctaaggaag ctaaaatgga gaaaaaaatc 360

actggatata ccaccgttga tatatcccaa tggcatcgta aagaacattt tgaggcattt 420

cagtcagttg ctcaatgtac ctataaccag accgttcagc tggatattac ggccttttta 480

aagaccgtaa agaaaaataa gcacaagttt tatccggcct ttattcacat tcttgcccgc 540

ctgatgaatg ctcatccgga atttcgtatg gcaatgaaag acggtgagct ggtgatatgg 600

gatagtgttc acccttgtta caccgttttc catgagcaaa ctgaaacgtt ttcatcgctc 660

tggagtgaat accacgacga tttccggcag tttctacaca tatattcgca agatgtggcg 720

tgttacggtg aaaacctggc ctatttccct aaagggttta ttgagaatat gtttttcgtc 780

tcagccaatc cctgggtgag tttcaccagt tttgatttaa acgtggccaa tatggacaac 840

ttcttcgccc ccgttttcac catgggcaaa tattatacgc aaggcgacaa ggtgctgatg 900

ccgctggcga ttcaggttca tcatgccgtt tgtgatggct tccatgtcgg cagaatgctt 960

aatgaattac aacagtactg cgatgagtgg cagggcgggg cgtaattttt ttaaggcagt 1020

tattggtgcc cttaaacgcc tggggtaatg actctctagc ttgaggcatc aaataaaacg 1080
aaaggctcag tcgaaagact gggcctttcg ttttatctgt tgtttgtcgg tgaacgctct 1140
cctgagtagg acaaatccgc cctctagatt acgtgcagtc gatgataagc tgtcaaacat 1200
gagaattgtg cctaatgagt gagctaactt acattaattg cgttgcgctc actgcccgct 1260
ttccagtcgg gaaacctgtc gtgccagctg cattaatgaa tcggccaacg cgcggggaga 1320
ggcggtttgc gtattgggcg ccagggtggt ttttcttttc accagtgaga cgggcaacag 1380
ctgattgccc ttcaccgcct ggccctgaga gagttgcagc aagcggtcca cgctggtttg 1440
ccccagcagg cgaaaatcct gtttgatggt ggttaacggc gggatataac atgagctgtc 1500
ttcggtatcg tcgtatccca ctaccgagat atccgcacca acgcgcagcc cggactcggt 1560
aatggcgcgc attgcgccca gcgccatctg atcgttggca accagcatcg cagtgggaac 1620
gatgccctca ttcagcattt gcatggtttg ttgaaaaccg gacatggcac tccagtcgcc 1680
ttcccgttcc gctatcggct gaatttgatt gcgagtgaga tatttatgcc agccagccag 1740
acgcagacgc gccgagacag aacttaatgg gcccgctaac agcgcgattt gctggtgacc 1800
caatgcgacc agatgctcca cgcccagtcg cgtaccgtct tcatgggaga aaataatact 1860
gttgatgggt gtctggtcag agacatcaag aaataacgcc ggaacattag tgcaggcagc 1920
ttccacagca atggcatcct ggtcatccag cggatagtta atgatcagcc cactgacgcg 1980
ttgcgcgaga agattgtgca ccgccgcttt acaggcttcg acgccgcttc gttctaccat 2040
cgacaccacc acgctggcac ccagttgatc ggcgcgagat ttaatcgccg cgacaatttg 2100
cgacggcgcg tgcagggcca gactggaggt ggcaacgcca atcagcaacg actgtttgcc 2160
cgccagttgt tgtgccacgc ggttgggaat gtaattcagc tccgccatcg ccgcttccac 2220
tttttcccgc gttttcgcag aaacgtggct ggcctggttc accacgcggg aaacggtctg 2280
ataagagaca ccggcatact ctgcgacatc gtataacgtt actggtttca cattcaccac 2340
cctgaattga ctctcttccg ggcgctatca tgccataccg cgaaaggttt tgcaccattc 2400
gatggtgtcg gaatttcggg cagcgttggg tcctggccac gggtgcgcat gatctagagc 2460
tgcctcgcgc gtttcggtga tgacggtgaa aacctctgac acatgcagct cccggagacg 2520
gtcacagctt gtctgtaagc ggatgccggg agcagacaag cccgtcaggg cgcgtcagcg 2580
ggtgttggcg ggtgtcgggg cgcagccatg acccagtcac gtagcgatag cggagtgtat 2640
actggcttaa ctatgcggca tcagagcaga ttgtactgag agtgcaccat atgcggtgtg 2700
aaataccgca cagatgcgta aggagaaaat accgcatcag gcgctcttcc gcttcctcgc 2760
tcactgactc gctgcgctcg gtcgttcggc tgcggcgagc ggtatcagct cactcaaagg 2820
cggtaatacg gttatccaca gaatcagggg ataacgcagg aaagaacatg tgagcaaaag 2880
gccagcaaaa ggccaggaac cgtaaaaagg ccgcgttgct ggcgtttttc cataggctcc 2940

gcccccctga cgagcatcac aaaaatcgac gctcaagtca gaggtggcga aacccgacag 3000
gactataaag ataccaggcg tttccccctg gaagctccct cgtgcgctct cctgttccga 3060
ccctgccgct taccggatac ctgtccgcct ttctcccttc gggaagcgtg gcgctttctc 3120
atagctcacg ctgtaggtat ctcagttcgg tgtaggtcgt tcgctccaag ctgggctgtg 3180
tgcacgaacc ccccgttcag cccgaccgct gcgccttatc cggtaactat cgtcttgagt 3240
ccaacccggt aagacacgac ttatcgccac tggcagcagc cactggtaac aggattagca 3300
gagcgaggta tgtaggcggt gctacagagt tcttgaagtg gtggcctaac tacggctaca 3360
ctagaaggac agtatttggt atctgcgctc tgctgaagcc agttaccttc ggaaaaagag 3420
ttggtagctc ttgatccggc aaacaaacca ccgctggtag cggtggtttt tttgtttgca 3480
agcagcagat tacgcgcaga aaaaaaggat ctcaagaaga tcctttgatc ttttctacgg 3540
ggtctgacgc tcagtggaac gaaaactcac gttaagggat tttggtcatg gcgttaaggg 3600
attttggtca tgaattaatt cttagaaaaa ctcatcgagc atcaaatgaa actgcaattt 3660
attcatatca ggattatcaa taccatattt ttgaaaaagc cgtttctgta atgaaggaga 3720
aaactcaccg aggcagttcc ataggatggc aagatcctgg tatcggtctg cgattccgac 3780
tcgtccaaca tcaatacaac ctattaattt cccctcgtca aaaataaggt tatcaagtga 3840
gaaatcacca tgagtgacga ctgaatccgg tgagaatggc aaaagtttat gcatttcttt 3900
ccagacttgt tcaacaggcc agccattacg ctcgtcatca aaatcactcg catcaaccaa 3960
accgttattc attcgtgatt gcgcctgagc gagacgaaat acgcgatcgc tgttaaaagg 4020
acaattacaa acaggaatcg aatgcaaccg gcgcaggaac actgccagcg catcaacaat 4080
attttcacct gaatcaggat attcttctaa tacctggaat gctgttttcc cggggatcgc 4140
agtggtgagt aaccatgcat catcaggagt acggataaaa tgcttgatgg tcggaagagg 4200
cataaattcc gtcagccagt ttagtctgac catctcatct gtaacatcat tggcaacgct 4260
acctttgcca tgtttcagaa acaactctgg cgcatcgggc ttcccataca atcgatagat 4320
tgtcgcacct gattgcccga cattatcgcg agcccattta tacccatata aatcagcatc 4380
catgttggaa tttaatcgcg gcctagagca agacgtttcc cgttgaatat ggctcataac 4440
accccttgta ttactgttta tgtaagcaga cagttttatt gttcatgacc atgacattaa 4500
cctataaaaa taggcgtatc acgaggccct ttcgtcttca c 4541

<210> 10
<211> 999
<212> DNA
<213> Artificial Sequence

<220>
<223> amplified PCR product

<400> 10
cggataacaa tttcacacag aggagaaatt aactatgaaa aaaactgcta tcgctatcgc 60

tgttgctctg gctggtttcg ctactgttgc tcaggccgaa ttcatgcgca gcagcctggt 120

gctgtttttt gtgagcgcgt ggaccgcgct ggcgagcccg attcgccgcg aagtgagcca 180

ggatctgttt aaccagttta acctgtttgc gcagtatagc gcggcggcgt attgcggcaa 240

aaacaacgat gcgccggcgg gcaccaacat tacctgcacc ggcaacgcgt gcccggaagt 300

ggaaaaagcg gatgcgacct ttctgtatag ctttgaagat agcggcgtgg gcgatgtgac 360

cggctttctg gcgctggata acaccaacaa actgattgtg ctgagctttc gcggcagccg 420

cagcattgaa aactggattg gcaacctgaa ctttgatctg aaagaaatta acgatatttg 480

cagcggctgc cgcggccatg atggctttac cagcagctgg cgcagcgtgg cggataccct 540

gcgccagaaa gtggaagatg cggtgcgcga acatccggat tatcgcgtgg tgtttaccgg 600

ccatagcctg ggcggcgcgc tggcgaccgt ggcgggcgcg gatctgcgcg gcaacggcta 660

tgatattgat gtgtttagct atggcgcgcc gcgcgtgggc aaccgcgcgt ttgcggaatt 720

tctgaccgtg cagaccggcg gcaccctgta tcgcattacc cataccaacg atattgtgcc 780

gcgcctgccg ccgcgcgaat ttggctatag ccatagcagc ccggaatatt ggattaaaag 840

cggcaccctg gtgccggtga cccgcaacga tattgtgaaa attgaaggca ttgatgcgac 900

cggcggcaac aaccagccga acattccgga tattccggcg catctgtggt attttggcct 960

gattggcacc tgcctgtgac gcatgcgagc tcggtaccc 999

<210> 11
<211> 5439
<212> DNA
<213> Artificial Sequence

<220>
<223> pQE80L-kan{PT5}[ompA-lipTl(co_Ec)]

<400> 11
cgggtcgacc tgcagccaag cttaattagc tgagcttgga ctcctgttga tagatccagt 60

aatgacctca gaactccatc tggatttgtt cagaacgctc ggttgccgcc gggcgttttt 120

tattggtgag aatccaagct agcttggcga gattttcagg agctaaggaa gctaaaatgg 180

agaaaaaaat cactggatat accaccgttg atatatccca atggcatcgt aaagaacatt 240

ttgaggcatt tcagtcagtt gctcaatgta cctataacca gaccgttcag ctggatatta 300

cggccttttt aaagaccgta aagaaaaata agcacaagtt ttatccggcc tttattcaca 360

ttcttgcccg cctgatgaat gctcatccgg aatttcgtat ggcaatgaaa gacggtgagc 420

tggtgatatg ggatagtgtt cacccttgtt acaccgtttt ccatgagcaa actgaaacgt 480

tttcatcgct ctggagtgaa taccacgacg atttccggca gtttctacac atatattcgc 540

aagatgtggc gtgttacggt gaaaacctgg cctatttccc taaagggttt attgagaata 600
tgtttttcgt ctcagccaat ccctgggtga gtttcaccag ttttgattta aacgtggcca 660
atatggacaa cttcttcgcc cccgttttca ccatgggcaa atattatacg caaggcgaca 720
aggtgctgat gccgctggcg attcaggttc atcatgccgt ttgtgatggc ttccatgtcg 780
gcagaatgct taatgaatta caacagtact gcgatgagtg gcagggcggg gcgtaatttt 840
tttaaggcag ttattggtgc ccttaaacgc ctggggtaat gactctctag cttgaggcat 900
caaataaaac gaaaggctca gtcgaaagac tgggcctttc gttttatctg ttgtttgtcg 960
gtgaacgctc tcctgagtag gacaaatccg ccctctagat tacgtgcagt cgatgataag 1020
ctgtcaaaca tgagaattgt gcctaatgag tgagctaact tacattaatt gcgttgcgct 1080
cactgcccgc tttccagtcg ggaaacctgt cgtgccagct gcattaatga atcggccaac 1140
gcgcggggag aggcggtttg cgtattgggc gccagggtgg tttttctttt caccagtgag 1200
acgggcaaca gctgattgcc cttcaccgcc tggccctgag agagttgcag caagcggtcc 1260
acgctggttt gccccagcag gcgaaaatcc tgtttgatgg tggttaacgg cgggatataa 1320
catgagctgt cttcggtatc gtcgtatccc actaccgaga tatccgcacc aacgcgcagc 1380
ccggactcgg taatggcgcg cattgcgccc agcgccatct gatcgttggc aaccagcatc 1440
gcagtgggaa cgatgccctc attcagcatt tgcatggttt gttgaaaacc ggacatggca 1500
ctccagtcgc cttcccgttc cgctatcggc tgaatttgat tgcgagtgag atatttatgc 1560
cagccagcca gacgcagacg cgccgagaca gaacttaatg ggcccgctaa cagcgcgatt 1620
tgctggtgac ccaatgcgac cagatgctcc acgcccagtc gcgtaccgtc ttcatgggag 1680
aaaataatac tgttgatggg tgtctggtca gagacatcaa gaaataacgc cggaacatta 1740
gtgcaggcag cttccacagc aatggcatcc tggtcatcca gcggatagtt aatgatcagc 1800
ccactgacgc gttgcgcgag aagattgtgc accgccgctt tacaggcttc gacgccgctt 1860
cgttctacca tcgacaccac cacgctggca cccagttgat cggcgcgaga tttaatcgcc 1920
gcgacaattt gcgacggcgc gtgcagggcc agactggagg tggcaacgcc aatcagcaac 1980
gactgtttgc ccgccagttg ttgtgccacg cggttgggaa tgtaattcag ctccgccatc 2040
gccgcttcca ctttttcccg cgttttcgca gaaacgtggc tggcctggtt caccacgcgg 2100
gaaacggtct gataagagac accggcatac tctgcgacat cgtataacgt tactggtttc 2160
acattcacca ccctgaattg actctcttcc gggcgctatc atgccatacc gcgaaaggtt 2220
ttgcaccatt cgatggtgtc ggaatttcgg gcagcgttgg gtcctggcca cgggtgcgca 2280
tgatctagag ctgcctcgcg cgtttcggtg atgacggtga aaacctctga cacatgcagc 2340
tcccggagac ggtcacagct tgtctgtaag cggatgccgg gagcagacaa gcccgtcagg 2400

gcgcgtcagc gggtgttggc gggtgtcggg gcgcagccat gacccagtca cgtagcgata 2460
gcggagtgta tactggctta actatgcggc atcagagcag attgtactga gagtgcacca 2520
tatgcggtgt gaaataccgc acagatgcgt aaggagaaaa taccgcatca ggcgctcttc 2580
cgcttcctcg ctcactgact cgctgcgctc ggtcgttcgg ctgcggcgag cggtatcagc 2640
tcactcaaag gcggtaatac ggttatccac agaatcaggg gataacgcag gaaagaacat 2700
gtgagcaaaa ggccagcaaa aggccaggaa ccgtaaaaag gccgcgttgc tggcgttttt 2760
ccataggctc cgcccccctg acgagcatca caaaaatcga cgctcaagtc agaggtggcg 2820
aaacccgaca ggactataaa gataccaggc gtttccccct ggaagctccc tcgtgcgctc 2880
tcctgttccg accctgccgc ttaccggata cctgtccgcc tttctccctt cgggaagcgt 2940
ggcgctttct catagctcac gctgtaggta tctcagttcg gtgtaggtcg ttcgctccaa 3000
gctgggctgt gtgcacgaac cccccgttca gcccgaccgc tgcgccttat ccggtaacta 3060
tcgtcttgag tccaacccgg taagacacga cttatcgcca ctggcagcag ccactggtaa 3120
caggattagc agagcgaggt atgtaggcgg tgctacagag ttcttgaagt ggtggcctaa 3180
ctacggctac actagaagga cagtatttgg tatctgcgct ctgctgaagc cagttacctt 3240
cggaaaaaga gttggtagct cttgatccgg caaacaaacc accgctggta gcggtggttt 3300
ttttgtttgc aagcagcaga ttacgcgcag aaaaaaagga tctcaagaag atcctttgat 3360
cttttctacg gggtctgacg ctcagtggaa cgaaaactca cgttaaggga ttttggtcat 3420
ggcgttaagg gattttggtc atgaattaat tcttagaaaa actcatcgag catcaaatga 3480
aactgcaatt tattcatatc aggattatca ataccatatt tttgaaaaag ccgtttctgt 3540
aatgaaggag aaaactcacc gaggcagttc cataggatgg caagatcctg gtatcggtct 3600
gcgattccga ctcgtccaac atcaatacaa cctattaatt tcccctcgtc aaaaataagg 3660
ttatcaagtg agaaatcacc atgagtgacg actgaatccg gtgagaatgg caaaagttta 3720
tgcatttctt tccagacttg ttcaacaggc cagccattac gctcgtcatc aaaatcactc 3780
gcatcaacca aaccgttatt cattcgtgat tgcgcctgag cgagacgaaa tacgcgatcg 3840
ctgttaaaag gacaattaca aacaggaatc gaatgcaacc ggcgcaggaa cactgccagc 3900
gcatcaacaa tattttcacc tgaatcagga tattcttcta atacctggaa tgctgttttc 3960
ccggggatcg cagtggtgag taaccatgca tcatcaggag tacggataaa atgcttgatg 4020
gtcggaagag gcataaattc cgtcagccag tttagtctga ccatctcatc tgtaacatca 4080
ttggcaacgc tacctttgcc atgtttcaga aacaactctg gcgcatcggg cttcccatac 4140
aatcgataga ttgtcgcacc tgattgcccg acattatcgc gagcccattt atacccatat 4200
aaatcagcat ccatgttgga atttaatcgc ggcctagagc aagacgtttc ccgttgaata 4260
tggctcataa cacccttgt attactgttt atgtaagcag acagttttat tgttcatgac 4320

catgacatta acctataaaa ataggcgtat cacgaggccc tttcgtcttc acctcgagaa 4380

atcataaaaa atttatttgc tttgtgagcg gataacaatt ataatagatt caattgtgag 4440

cggataacaa tttcacacag aggagaaatt aactatgaaa aaaactgcta tcgctatcgc 4500

tgttgctctg gctggtttcg ctactgttgc tcaggccgaa ttcatgcgca gcagcctggt 4560

gctgtttttt gtgagcgcgt ggaccgcgct ggcgagcccg attcgccgcg aagtgagcca 4620

ggatctgttt aaccagttta acctgtttgc gcagtatagc gcggcggcgt attgcggcaa 4680

aaacaacgat gcgccggcgg gcaccaacat tacctgcacc ggcaacgcgt gcccggaagt 4740

ggaaaaagcg gatgcgacct ttctgtatag ctttgaagat agcggcgtgg gcgatgtgac 4800

cggctttctg gcgctggata acaccaacaa actgattgtg ctgagctttc gcggcagccg 4860

cagcattgaa aactggattg gcaacctgaa ctttgatctg aaagaaatta acgatatttg 4920

cagcggctgc cgcggccatg atggctttac cagcagctgg cgcagcgtgg cggataccct 4980

gcgccagaaa gtggaagatg cggtgcgcga acatccggat tatcgcgtgg tgtttaccgg 5040

ccatagcctg ggcggcgcgc tggcgaccgt ggcgggcgcg gatctgcgcg gcaacggcta 5100

tgatattgat gtgtttagct atggcgcgcc gcgcgtgggc aaccgcgcgt ttgcggaatt 5160

tctgaccgtg cagaccggcg gcaccctgta tcgcattacc cataccaacg atattgtgcc 5220

gcgcctgccg ccgcgcgaat ttggctatag ccatagcagc ccggaatatt ggattaaaag 5280

cggcaccctg gtgccggtga cccgcaacga tattgtgaaa attgaaggca ttgatgcgac 5340

cggcggcaac aaccagccga acattccgga tattccggcg catctgtggt attttggcct 5400

gattggcacc tgcctgtgac gcatgcgagc tcggtaccc 5439

<210> 12
<211> 314
<212> PRT
<213> Artificial Sequence

<220>
<223> the fusion-protein ompA-LipTl

<400> 12

Met Lys Lys Thr Ala Ile Ala Ile Ala Val Ala Leu Ala Gly Phe Ala
1               5                   10                  15

Thr Val Ala Gln Ala Glu Phe Met Arg Ser Ser Leu Val Leu Phe Phe
            20                  25                  30

Val Ser Ala Trp Thr Ala Leu Ala Ser Pro Ile Arg Arg Glu Val Ser
        35                  40                  45

Gln Asp Leu Phe Asn Gln Phe Asn Leu Phe Ala Gln Tyr Ser Ala Ala

50 55 60

Ala Tyr Cys Gly Lys Asn Asn Asp Ala Pro Ala Gly Thr Asn Ile Thr
65 70 75 80

Cys Thr Gly Asn Ala Cys Pro Glu Val Glu Lys Ala Asp Ala Thr Phe
85 90 95

Leu Tyr Ser Phe Glu Asp Ser Gly Val Gly Asp Val Thr Gly Phe Leu
100 105 110

Ala Leu Asp Asn Thr Asn Lys Leu Ile Val Leu Ser Phe Arg Gly Ser
115 120 125

Arg Ser Ile Glu Asn Trp Ile Gly Asn Leu Asn Phe Asp Leu Lys Glu
130 135 140

Ile Asn Asp Ile Cys Ser Gly Cys Arg Gly His Asp Gly Phe Thr Ser
145 150 155 160

Ser Trp Arg Ser Val Ala Asp Thr Leu Arg Gln Lys Val Glu Asp Ala
165 170 175

Val Arg Glu His Pro Asp Tyr Arg Val Val Phe Thr Gly His Ser Leu
180 185 190

Gly Gly Ala Leu Ala Thr Val Ala Gly Ala Asp Leu Arg Gly Asn Gly
195 200 205

Tyr Asp Ile Asp Val Phe Ser Tyr Gly Ala Pro Arg Val Gly Asn Arg
210 215 220

Ala Phe Ala Glu Phe Leu Thr Val Gln Thr Gly Gly Thr Leu Tyr Arg
225 230 235 240

Ile Thr His Thr Asn Asp Ile Val Pro Arg Leu Pro Pro Arg Glu Phe
245 250 255

Gly Tyr Ser His Ser Ser Pro Glu Tyr Trp Ile Lys Ser Gly Thr Leu
260 265 270

Val Pro Val Thr Arg Asn Asp Ile Val Lys Ile Glu Gly Ile Asp Ala
275 280 285

Thr Gly Gly Asn Asn Gln Pro Asn Ile Pro Asp Ile Pro Ala His Leu
290 295 300

Trp Tyr Phe Gly Leu Ile Gly Thr Cys Leu
305 310

<210> 13
<211> 230
<212> PRT
<213> Artificial Sequence

<220>
<223> AlkL mod1

<400> 13

Val Ser Phe Ser Asn Tyr Lys Val Ile Ala Met Pro Val Leu Val Ala
1 5 10 15

Asn Phe Val Leu Gly Ala Ala Thr Ala Trp Ala Asn Glu Asn Tyr Pro
20 25 30

Ala Lys Ser Ala Gly Tyr Asn Gln Gly Asp Trp Val Ala Ser Phe Asn
35 40 45

Phe Ser Lys Val Tyr Val Gly Glu Glu Leu Gly Asp Leu Asn Val Gly
50 55 60

Gly Gly Ala Leu Pro Asn Ala Asp Val Ser Ile Gly Asn Asp Thr Thr
65 70 75 80

Leu Thr Phe Asp Ile Ala Tyr Phe Val Ser Ser Asn Ile Ala Val Asp
85 90 95

Phe Phe Val Gly Val Pro Ala Arg Ala Lys Phe Gln Gly Glu Lys Ser
100 105 110

Ile Ser Ser Leu Gly Arg Val Ser Glu Val Asp Tyr Gly Pro Ala Ile
115 120 125

Leu Ser Leu Gln Tyr His Tyr Asp Ser Phe Glu Arg Leu Tyr Pro Tyr
130 135 140

Val Gly Val Gly Val Gly Arg Val Leu Phe Phe Asp Lys Thr Asp Gly
145 150 155 160

Ala Leu Ser Ser Phe Asp Ile Lys Asp Lys Trp Ala Pro Ala Phe Gln
165 170 175

Val Gly Leu Arg Tyr Asp Leu Gly Asn Ser Trp Met Leu Asn Ser Asp
180 185 190

Val Arg Tyr Ile Pro Phe Lys Thr Asp Val Thr Gly Thr Leu Gly Pro

```
            195                 200                 205

Val Pro Val Ser Thr Lys Ile Glu Val Asp Pro Phe Ile Leu Ser Leu
    210                 215                 220

Gly Ala Ser Tyr Val Phe
225                 230
```

## Description

### FIELD OF THE INVENTION

The present invention relates to biotechnological methods and cells for producing at least one acyl amino acid from oils.

### BACKGROUND OF THE INVENTION

Acyl amino acids are a class of surface-active agents with a variety of uses, for example as detergents for washing purposes, emulsifiers in food products and as essential ingredients in various personal care products such as shampoos, soaps, moisturizing agents and the like. In addition to having both hydrophobic and hydrophilic regions, a prerequisite for use as a surfactant, requires these surfactants to be made of naturally occurring molecules. These naturally occurring molecules include amino acids and fatty acids, which are not only non-hazardous and environmentally acceptable but may be readily produced at a large scale using inexpensive biological raw materials. Acyl amino acids may also be used in pharmacological research as neuromodulators and/or probes for new drug targets.

Traditionally acyl amino acids have been produced at an industrial scale starting with materials derived from tropical oils. More specifically, activated fatty acids provided in the form of acid chlorides have been used to acylate amino acids in an aqueous alkaline medium as described in GB 1 483 500. Shortcomings of such approaches include the need to add hazardous chemicals such as sulphuric acid or anhydrides thereof. Other synthetic approaches are associated with the accumulation of by-products such as chloride salts which have undesirable effects on surfactancy. For example, Schotten-Baumann synthesis is an example of a synthesis route that is commonly used for the production of acyl amino acids. However, this method has the disadvantage that chlorinated fatty acids are used in the method and these fatty acids are synthesized via toxic chemicals like phosgene or PCL3.

A range of biotechnological routes towards production of acyl amino acids has been described. However, none of them is adequate for the commercial large-scale production of acyl amino acids owing to low yields, insufficient purities and the need for multi-step purification procedures. In particular, only a small proportion of the carbon substrates fed to biotechnologically useful organisms is actually converted to acyl amino acids, whilst much of these substrates is consumed by reactions of the primary metabolism.

Another problem associated with biotechnological routes is the fact that a mixture of products is obtained and thus the composition is difficult to control. More specifically, a range of fatty acids may be converted to acyl amino acids, even though production of a single adduct may be desirable. This also leads to foaming which makes controlling the reaction difficult. Since the mixture comprises compounds highly related in terms of chemical structure, purifying or at least enriching a single component in an efficient and straightforward manner is usually beyond technical feasibility.

Accordingly, there is a need in the art for a means of producing acyl amino acids more efficiently and at lower costs.

### DESCRIPTION OF THE INVENTION

The present invention attempts to solve the problems above by providing at least one cell and/or method of producing at least one acyl amino acid from any naturally existing oil. This is advantageous as it allows for the use of naturally occurring oil instead of fatty acids. An additional hydrolysis step for the preparation of free fatty acids may thus be avoided. This allows the process of acyl amino acid production to be sped up and also saves cost. Further, the use of oils as a substrate for acyl amino acid production may be advantageous as the oil results in a less foamy effect and therefore less or no anti foaming agent needs to be used in the method according to any aspect of the present invention.

According to one aspect of the present invention there is provided a microbial cell for producing at least one acyl amino acid, wherein the cell is genetically modified to comprise:

- a first genetic mutation that increases the expression relative to a wild type cell of at least one lipase (EC 3.1.1) ($E_1$) capable of hydrolysing at least one glyceride to at least one fatty acid; and
- a second and a third genetic mutation that increases the expression relative to a wild type cell of:

  (i) an amino acid-N-acyl-transferase (EC 2.3.2) ($E_2$), and
  (ii an acyl-CoA synthetase (EC 6.2.1.1) ($E_3$) respectively

  that enables the cell to convert the fatty acid to at least one acyl amino acid and wherein the cell has a reduced fatty acid degradation capacity.

The cell according to any aspect of the present invention may be capable of converting at least one natural oil to free fatty acid(s) and the cell may further comprise a second and a third genetic mutation that enables the cell to produce at least one acyl amino acid from the fatty acid. In particular, the first genetic mutation may enable to cell to convert a naturally occurring and cheap source of fats to a fatty acid that may then be used as a suitable raw material for the production of more useful higher organic compounds such as acyl amino acids. More in particular, the first genetic mutation may be of at least one lipase that converts glycerides that are found in natural oils to fatty acids. The glyceride may be a monoglyceride, diglyceride or triglyceride. In particular, the glyceride used according to any aspect of the present invention may be a triglyceride.

The terms "natural oils," may refer to oils derived from plants or animal sources. The term "natural oil" includes natural oil derivatives. Natural oils may also include modified plant or animal sources (e.g., genetically modified plant or animal sources). Examples of natural oils include, but are not limited to, vegetable oils, algae oils, fish oils, animal fats, tall oils, derivatives of these oils, combinations of any of these oils, and the like. Non-limiting examples of vegetable oils include canola oil, rapeseed oil, coconut oil, corn oil, cottonseed oil, olive oil, palm oil, peanut oil, safflower oil, sesame oil, soybean oil, sunflower oil, linseed oil, palm kernel oil, tung oil, jatropha oil, mustard oil, pennycress oil, camelina oil, and castor oil. Natural oils may also include animal fats such as lard, tallow, poultry fat, yellow grease, and fish oil. Tall oils are by-products of wood pulp manufacture. Any one of these natural oils may be used as a source of glycerides according to any aspect of the present invention.

Lipases are enzymes that catalyse hydrolysis reactions to decompose oils into free fatty acids and glycerol. Various types of animals, plants and microorganisms are known to have lipases. Differences in the activity of lipases occur due to bonding position within the glyceride, carbon chain length of the fatty acid, number of double bonds, and the like. It is thus possible to selectively hydrolyse fatty acids using such lipases, and as a result, it becomes possible to concentrate a specific fatty acid within the glyceride fraction. For example, when a lipase produced by a kind of the genus *Candida* is used, it is known that hydrolysis reaction of fish oil results in concentration of highly unsaturated fatty acids, such as docosahexaenoic acid, in the undecomposed glyceride fraction. Similarly, the suitable lipase may be selected to be used to hydrolyse the relevant oils to the desired fatty acid and glycerol. In one example, the lipase used according to any aspect of the present invention may be derived from a microorganism selected from the group consisting of: *Candida cylindracea, Alcaligenes sp., Burkholderia cepacia, Pseudomonas fluorescens, Thermomyces lanuginosus, Rhizomucor miehei,* and *Pseudomonas sp.* In particular, the lipase may be from *Thermomyces lanuginosus.* Such lipases include lipases obtained from microorganisms belonging to *Alcaligenes sp.* (Lipase QLM, Lipase QLC, Lipase PL, all produced by Meito Sangyo Co., Ltd.), lipases obtained from microorganisms belonging to *Burkholderia cepacia* (Lipase PS, produced by Amano Enzyme Inc.), lipases obtained from microorganisms belonging to *Pseudomonas fluorescens* (Lipase AK, produced by Amano Enzyme Inc.), lipases obtained from microorganisms belonging to *Thermomyces lanuginosus* (Lipozyme TLIM, produced by Novozymes), or the like. Lipases derived from *Candia rugose, Rhizomucor miehei, Pseudomonas sp.* and the like. All of these lipases are commercially marketed and may be readily obtained. As may be required, these lipases may be immobilized prior to use.

More in particular, the lipase ($E_1$) used according to any aspect of the present invention may comprise a sequence of 50%, 55%, 60%, 65%, 70%, 75%, 80%, 85%, 90%, 95%, 100% sequence identity with SEQ ID NO:4.

The second and third genetic mutation in the cell may be in at least one amino acid-N-acyl-transferase ($E_2$) and (ii) acyl-CoA synthetase ($E_3$) respectively. In another example, the second genetic mutation may result in increased activity relative to the wild type cell of glycine N-acyl transferase ($E_{2a}$). In yet another example, the cell according to any aspect of the present invention may be genetically modified to have a mutation in an enzyme that is involved in a lipase ($E_1$) and a mutation in an enzyme that may result in increased activity relative to the wild type cell of glycine-N-acyl transferase ($E_{2a}$).

According to any aspect of the present invention, the second and third genetic mutation may result in the cell having increased expression of (i) amino acid-N-acyl-transferase ($E_2$) and (ii) acyl-CoA synthetase ($E_3$) respectively. The combination of amino acid-N-acyl transferase and an acyl-CoA synthetase, expressed according to any aspect of the present invention may be used to convert a variety of fatty acids and/or amino acids including a mixture comprising unsaturated and saturated fatty acids, to acyl amino acids. In particular, the amino acid-N-acyl transferases may be used to convert short unsaturated fatty acids such as lauroleic acid to an acyl amino acid. More in particular, the amino acid-N-acyl-transferase may be capable of converting a variety of fatty acids including short unsaturated fatty acids such as lauroleic acid to an acyl amino acid with an increase in the yields of acyl amino acids produced. Further, the composition of acyl amino acids produced in a cell, more specifically the length of fatty acids incorporated into such acyl amino acids, may be controlled by introducing into the cell one or more specific acyl-CoA thioesterases or altering the expression of one or more acyl-CoA thioesterases endogenously expressed by the cell.

The cell according to any aspect of the present invention may comprise a further genetic mutation that enables the cell to have increased expression of acyl-CoA thioesterase ($E_5$). The term "acyl-CoA thioesterase", as used herein, refers to an enzyme capable of hydrolysing acyl-CoA. In particular, the acyl-CoA thioesterase comprises a sequence selected from the group consisting of AEM72521.1 and AAC49180.1 or a variant thereof. The nucleotide sequence of E5 may be SEQ ID NO:3. The activity of acyl-CoA thioesterase may be assayed using various assays described in the state of the art. Briefly, the reaction of Ellman's reagent, which reacts with free thiol groups associated with CoASH formed upon hydrolysis of acyl-CoA may be detected by spectophotometrically monitoring absorbance at 412 nm.

The term "amino acid-N-acyl transferase", as used herein, refers to an enzyme capable of catalysing the conversion of acyl-CoA, for example the CoA ester of an acid, and an amino acid, for example a proteinogenic amino acid, for example glycine, glutamic acid, and/or any other amino acid to an acyl amino acid. Suitable amino acid-N-acyl transferases have been described in the prior art, for example in Waluk, D. P., Schultz, N., and Hunt, M. C. (2010). In particular, the amino acid sequence of amino acid-N-acyl transferase may be selected from the group consisting of SEQ ID NO:1, NP_659453.3 NP_001010904.1, , XP_001147054.1, AAH16789.1, AA073139.1, XP_003275392.1, XP_002755356.1, XP_003920208.1, XP_004051278.1, XP_006147456.1, XP_006214970.1, XP_003801413.1, XP_006189704.1, XP_003993512.1, XP_005862181.1, XP_007092708.1, XP_006772167.1, XP_006091892.1, XP_005660936.1, XP_005911029.1, NP_001178259.1, XP_004016547.1, XP_005954684.1, ELR45061.1, XP_005690354.1, XP_004409352.1, XP_007519553.1, XP_004777729.1, XP_005660935.1, XP_004824058.1, XP_006068141.1, XP_006900486.1, XP_007497585.1, XP_002821801.2, XP_007497583.1, XP_003774260.1, XP_001377648.2, XP_003909843.1, XP_003801448.1, XP_001091958.1, XP_002821798.1, XP_005577840.1, XP_001092197.1, NP_001207423.1, NP_001207425.1, XP_003954287.1, NP_001271595.1, XP_003909848.1, XP_004087850.1, XP_004051279.1, XP_003920209.1, XP_005577835.1, XP_003774402.1, XP_003909846.1, XP_004389401.1, XP_002821802.1, XP_003774401.1, XP_007497581.1, EHH21814.1, XP_003909845.1, XP_005577839.1, XP_003774403.1, XP_001092427.1, XP_003275395.2, NP_542392.2, XP_001147271.1, XP_005577837.1, XP_003826420.1, XP_004051281.1, XP_001147649.2, XP_003826678.1, XP_003909847.1, XP_004682812.1, XP_004682811.1, XP_003734315.1, XP_004715052.1, BAG62195.1, XP_003777804.1, XP_003909849.1, XP_001092316.2, XP_006167891.1, XP_540580.2, XP_001512426.1, EAW73833.1, XP_003464217.1, XP_007519551.1, XP_003774037.1, XP_005954680.1, XP_003801411.1, NP_803479.1, XP_004437460.1, XP_006875830.1, XP_004328969.1, XP_004264206.1, XP_004683490.1, XP_004777683.1, XP_005954681.1, XP_003480745.1, XP_004777682.1, XP_004878093.1, XP_007519550.1, XP_003421399.1, EHH53167.1, XP_006172214.1 ,XP_003993453.1 ,AAI12537.1, XP_006189705.1 ,Q2KIR7.2, XP_003421465.1, NP_001009648.1, XP_003464328.1, XP_001504745.1, ELV11036.1, XP_005690351.1, XP_005216632.1, EPY77465.1, XP_005690352.1, XP_004016544.1, XP_001498276.2, XP_004264205.1, XP_005690353.1, XP_005954683.1, XP_004667759.1, XP_004479306.1, XP_004645843.1, XP_004016543.1, XP_002928268.1, XP_006091904.1, XP_005331614.1, XP_007196549.1, XP_007092705.1, XP_004620532.1, XP_004869789.1, EHA98800.1, XP_004016545.1, XP_004479307.1, XP_004093105.1, NP_001095518.1, XP_005408101.1, XP_004409350.1, XP_001498290.1, XP_006056693.1, XP_005216639.1, XP_007455745.1, XP_005352049.1, XP_004328970.1, XP_002709220.1, XP_004878092.1, XP_007196553.1, XP_006996816.1, XP_005331615.1, XP_006772157.1, XP_007196552.1, XP_004016546.1, XP_007628721.1, NP_803452.1, XP_004479304.1, DAA21601.1, XP_003920207.1, XP_006091906.1, XP_003464227.1, XP_006091903.1, XP_006189706.1, XP_007455744.1, XP_004585544.1, XP_003801410.1, XP_007124812.1, XP_006900488.1, XP_004777680.1, XP_005907436.1, XP_004389356.1, XP_007124811.1, XP_005660937.1, XP_007628724.1, XP_003513512.1, XP_004437813.1, XP_007628723.1, ERE78858.1, EPQ15380.1, XP_005862178.1, XP_005878672.1, XP_540581.1, XP_002928267.1, XP_004645845.1, EPQ05184.1, XP_003513511.1, XP_006214972.1, XP_007196545.1, XP_007196547.1, XP_006772160.1, XP_003801409.1, NP_001119750.1, XP_003801412.1, XP_006772159.1, EAW73832.1, XP_006091897.1, XP_006772163.1, XP_006091898.1, XP_005408105.1, XP_006900487.1, XP_003993454.1, XP_003122754.3, XP_007455746.1, XP_005331618.1, XP_004585337.1, XP_005063305.1, XP_006091895.1, XP_006772156.1, XP_004051276.1, XP_004683488.1, NP_666047.1, NP_001013784.2, XP_006996815.1, XP_006996821.1, XP_006091893.1, XP_006173036.1, XP_006214971.1, EPY89845.1, XP_003826423.1, NP_964011.2, XP_007092707.1, XP_005063858.1, BAL43174.1, XP_001161154.2, XP_007124813.1, NP_083826.1XP_003464239.1, XP_003275394.1, ELK23978.1, XP_004878097.1, XP_004878098.1, XP_004437459.1, XP_004264204.1, XP_004409351.1, XP_005352047.1, Q5RFP0.1, XP_005408107.1, XP_007659164.1, XP_003909852.1, XP_002755355.1, NP_001126806.1, AAP92593.1, NP_001244199.1, BAA34427.1, XP_005063859.1, NP_599157.2, XP_004667761.1, XP_006900489.1, XP_006215013.1, XP_005408100.1, XP_007628718.1, XP_003514769.1, XP_006160935.1, XP_004683489.1, XP_003464329.1, XP_004921258.1, XP_003801447.1, XP_006167892.1, XP_004921305.1, AAH89619.1, XP_004706162.1, XP_003583243.1, EFB16804.1, XP_006728603.1, EPQ05185.1, XP_002709040.1, XP_006875861.1, XP_005408103.1, XP_004391425.1, EDL41477.1, XP_006772158.1, EGW06527.1, AAH15294.1, XP_006772162.1, XP_005660939.1, XP_005352050.1, XP_006091901.1, XP_005878675.1, XP_004051323.1, EHA98803.1, XP_003779925.1, EDM12924.1, XP_003421400.1, XP_006160939.1, XP_006160938.1, XP_006160937.1, XP_006160936.1, XP_005702185.1, XP_005313023.1, XP_003769190.1, XP_002714424.1, XP_004715051.1, XP_007661593.1, XP_004590594.1, ELK23975.1, XP_004674085.1, XP_004780477.1, XP_006231186.1, XP_003803573.1, XP_004803176.1, EFB16803.1, XP_006056694.1, XP_005441626.1, XP_005318647.1XP_004605904.1, XP_005862182.1, XP_003430682.1, XP_004780478.1, XP_005239278.1, XP_003897760.1, XP_007484121.1, XP_004892683.1, XP_004414286.1, XP_006927013.1, XP_003923145.1, XP_852587.2, AAP97178.1, EHH53105.1, XP_005408113.1, XP_002915474.1, XP_005377590.1, XP_527404.2, XP_005552830.1, XP_004044211.1, NP_001180996.1, XP_003513513.2, XP_001498599.2, XP_002746654.1, XP_005072349.1, XP_006149181.1, EAX04334.1, XP_003833230.1, XP_005216635.1, XP_003404197.1, XP_007523363.1, XP_007433902.1, XP_003254235.1, XP_004471242.1, XP_005216634.1, XP_006860675.1, XP_004771956.1, XP_006038833.1, NP_001138534.1, XP_007068532.1, XP_003510714.1, ERE87950.1, XP_003986313.1, XP_006728644.1, XP_004878099.1, XP_003468014.1, XP_007095614.1, XP_004648849.1, XP_004869795.1, XP_004018927.1, XP_005696454.1, XP_006201985.1, XP_005960697.1, XP_004813725.1, XP_005496926.1, ELR45088.1, XP_004696625.1, XP_005860982.1, XP_005911003.1, XP_006260162.1, EPQ04414.1, XP_006099775.1, NP_001138532.1, XP_006190795.1, XP_004649775.1, XP_004424497.1, XP_004390885.1, XP_005911004.1, XP_003777803.1, XP_004312259.1, XP_005529140.1, XP_005314582.1, XP_006926523.1, XP_006926522.1, XP_004683491.1, XP_003826680.1, XP_003215018.1, XP_003215087.1, EGW12611.1, XP_006113023.1, XP_006882182.1, XP_007425200.1, XP_006041342.1, NP_001138533.1, EMP27694.1, XP_007497753.1, XP_006034252.1, or a variant thereof. Throughout this disclosure, any data base code, unless specified to the contrary, refers to a sequence available from the NCBI data bases, more specifically the version online on 5$^{th}$ August 2013, and comprises, if such sequence is a nucleotide sequence, the polypeptide sequence obtained by translating the former.

In particular, the first genetic mutation may increase the activity of glycine-N-acyl transferase ($E_{2a}$) and acyl-CoA synthetase ($E_3$) relative to the wild type cell. More in particular, the sequence of glycine-N-acyl transferase ($E_{2a}$) used according to any aspect of the present invention may have 50%, 55%, 60%, 65%, 70%, 75%, 80%, 85%, 90%, 95%, 100% sequence identity with SEQ ID NO:1 and/or the sequence of acyl-CoA synthetase ($E_3$) used according to any aspect of the present invention may have 50%, 55%, 60%, 65%, 70%, 75%, 80%, 85%, 90%, 95%, 100% sequence identity with SEQ ID NO:2.

In one example, the glycine-N-acyl transferase ($E_{2a}$) may be capable of producing N-acyl glutamate. In this example, the glycine-N-acyl transferase ($E_{2a}$) may comprise 50%, 55%, 60%, 65%, 70%, 75%, 80%, 85%, 90%, 95%, 100% sequence identity with SEQ ID NO:1.

The cell according to any aspect of the present invention may comprise a genetic mutation in a second amino acid acyl transferase that may be capable of working on other amino acids or variants thereof in conjunction with the activity of the lipase.

The acyl-CoA substrate consumed in the cell according to any aspect of the present invention may be purified from a cell, chemically synthesised or produced using an acyl-CoA synthetase. The term "acyl-CoA synthetase", as used herein, refers to an enzyme capable of catalysing the ATP- or GTP-dependent conversion of a fatty acid and CoA or ACP to acyl-CoA or acyl-ACP. In one example, the acyl-CoA synthetase may comprise amino acid sequence SEQ ID NO:2.

According to any aspect of the present invention, the term 'acyl-CoA synthetase' may refer to an acyl-CoA/ACP synthetase that may be capable of producing acyl thioester, i.e. acyl-CoA or acyl-ACP and/or catalysing the following reaction:

$$\text{fatty acid + CoA/ACP + ATP/GTP} \rightarrow \text{acyl-CoA/ACP + ADP/GDP + Pi}$$

Examples of acyl-CoA synthetases may include EC 6.2.1.3, EC 6.2.1.10, EC 6.2.1.15, EC 6.2.1.20 and the like. The state of the art describes various methods to detect acyl-CoA synthetase activity. For example, the activity of an acyl-CoA synthetase may be assayed as follows: the standard reaction mixture for the spectrophotometric assay (total volume, 1 ml) is composed of 0.1 M Tris-HC1 buffer pH 8.0, 1.6 mM Triton X-100, 5 mM dithiothreitol, 0.15 M KCl, 15 mM $MgCl_2$, 10 mM ATP, 0.1 mM potassium palmitate, 0.6 mM CoA, 0.2 mM potassium phosphoenolpyruvate, 0.15 mM NADH, 45 pg adenylate kinase per mL, 30 pg pyruvate kinase per mL and 30 pg lactate dehydrogenase per mL. The oxidation of NADH at 334 nm is followed with a recording spectrophotometer.

Alternatively, the activity of an acyl-CoA synthetase may be assayed as described in the state of the art, for example Kang, Y., et al, 2010. Briefly, the amount of free thiol in the form of unreacted CoASH may be determined by adding Ellmann's reagent and spectrophotometrically monitoring the absorbance at 410 nm, in a reaction buffer comprising 150 mM Tris-HCl (pH 7.2), 10 mM $MgCl_2$, 2 mM EDTA, 0.1 % Triton X-100, 5 mM ATP, 0.5 mM Coenzyme A (CoASH) and a fatty acid (30 to 300 mM).

Various acyl-CoA synthetases have been described in the state of the art, for example YP_001724804.1, WP_001563489.1 and NP_707317.1. In one example, the acyl-CoA synthetase comprises SEQ ID NO: 2, YP_001724804.1, BAA15609.1 or a variant thereof.

The cell according to any aspect of the present invention may be genetically different from the wild type cell. The genetic difference between the cell according to any aspect of the present invention and the wild type cell may be in the presence of a complete gene, amino acid, nucleotide etc. in the cell according to any aspect of the present invention that may be absent in the wild type cell. In one example, the cell according to any aspect of the present invention may comprise enzymes that enable the cell to produce at least one acyl amino acid and cell may further comprise at least one mutation that results in the conversion of naturally occurring oils to at least one fatty acid and glycerol. The wild type cell relative to the cell according to any aspect of the present invention may have none or no detectable activity of the enzymes that enable the cell according to any aspect of the present invention to produce at least one acyl amino acid; and the wild type cell may not be capable of hydrolysing oils to fatty acids as per normal.

The phrase "wild type" as used herein in conjunction with a cell may denote a cell with a genome make-up that is in a form as seen naturally in the wild. The term may be applicable for both the whole cell and for individual genes. The term "wild type" therefore does not include such cells or such genes where the gene sequences have been altered at least partially by man using recombinant methods.

A skilled person would be able to use any method known in the art to genetically modify a cell. According to any aspect of the present invention, the cell may be genetically modified so that in a defined time interval, within 2 hours, in particular within 8 hours or 24 hours, it forms at least twice, especially at least 10 times, at least 100 times, at least 1000 times or at least 10000 times more acyl amino acids than the wild-type cell. The increase in product formation can be determined for example by cultivating the cell according to any aspect of the present invention and the wild-type cell each separately under the same conditions (same cell density, same nutrient medium, same culture conditions) for a specified time interval in a suitable nutrient medium and then determining the amount of target product (acyl amino acid) in the nutrient medium.

The phrase 'decreased activity of an enzyme' and like may refer to a genetic modification that may be present in the cell according to any aspect of the present invention to decrease a specific enzymatic activity and this may be done by a gene disruption or a genetic modification. In particular, the decrease in activity of an enzyme relative to the wild type cell may be a 10, 15, 20, 25, 30, 35, 40, 45, 50, 55, 60, 65, 70, 75, 80, 85, 90, 95 or 100% less than the wild type cell.

The phrase "increased activity of an enzyme", as used herein is to be understood as increased intracellular activity. Basically, an increase in enzymatic activity can be achieved by increasing the copy number of the gene sequence or gene sequences that code for the enzyme, using a strong promoter, increasing translation by improved ribosome binding sites or optimized codon usage or employing a gene or allele that code for a corresponding enzyme with increased activity and optionally by combining these measures. Genetically modified cells used according to any aspect of the present invention are for example produced by transformation, transduction, conjugation or a combination of these methods with a vector that contains the desired gene, an allele of this gene or parts thereof and a vector that makes expression of the gene possible. Heterologous expression is in particular achieved by integration of the gene or of the alleles in the chromosome of the cell or an extra-chromosomally replicating vector. In particular, an increase in an activity of an enzyme relative to the wild type cell may be a 10, 15, 20, 25, 30, 35, 40, 45, 50, 55, 60, 65, 70, 75, 80, 85, 90, 95 or 100% more than the wild type cell.

In one example, the cell according to any aspect of the present invention may comprise a second and third genetic mutation that results in the overexpression of at least the enzymes amino acid-N-acyl transferase ($E_2$) and acyl-CoA synthetase ($E_3$). In particular, the cell may over express enzymes glycine N-acyl transferase ($E_{2a}$) and acyl-CoA/ACP synthetase ($E_3$). The expression of glycine-N-acyl transferase may be measured using at least the assay disclosed in Badenhorst CP, 2012. Namely, DTNB, water and cell lysate are mixed together and incubated at 37°C for 10 min while monitoring absorbance at 412 nm.

In particular, the amino acid-N-acyl-transferase ($E_2$) may be a human glycine N-acyl-transferase ($E_{2a}$). In one example, $E_1$ may comprise SEQ ID NO:4 or a variant thereof. In another example, $E_2$ may comprise SEQ ID NO:1 and the acyl-CoA synthetase ($E_3$) may comprise SEQ ID NO: 2 or a variant thereof. More in particular, the cell according to any aspect of the present invention may comprise a first genetic mutation in $E_1$ which may comprise SEQ ID NO:4 and a second and third genetic mutation in $E_1$ and $E_2$, wherein $E_1$ may comprise a amino acid sequence of SEQ ID NO: 1 and $E_2$ may comprise amino acid sequence of SEQ ID NO: 2.

According to any aspect of the present invention, a formula referring to a chemical group that represents the dissociated or undissociated state of a compound capable of dissociating in an aqueous solution comprises both the dissociated and the undissociated state and the various salt forms of the group. For example, the residue -COOH comprises both the protonated (-COOH) as well as the unprotonated ($-COO^-$) carboxylic acid.

The term "acyl amino acid", as used herein, refers to the product of the reaction catalysed by an amino acid-N-acyl transferase, a compound represented by the formula acyl-CO-NH-CHR-COOH, wherein R is the side chain of a proteinogenic amino acid, and wherein the term "acyl" refers to the acyl residue of a fatty acid. In one example, the term "fatty acid", as used herein, means a carboxylic acid, for example an alkanoic acid, with at least 6, 8, 10, or 12 carbon atoms. In one example, it is a linear fatty acid, in another example, it is branched. In one example it is a saturated fatty acid. In another example, it is unsaturated. In one example, it is a fatty acid with 6, 7, 8, 9, 10, 11, 12, 13, 14, 15, 16, 17, 18, 19, 20, 21, 22, 23, 24, 25, 26, 27, 28, 29, 30 carbon atoms. In particular, the acyl amino acid may be N-acyl glycinate, N-acyl glutamate, N-acyl Alaninate, N-acyl Sarcosinate, N-acyl Arginate, N-acyl Lysinate, N-acyl Threoninate, N-acyl Prolinate and the like. In one example, the acyl amino acid produced according to any aspect of the present invention may be selected from the group consisting of cocoyl glycinate, cocoyl glutamate, cocoyl alaninate, cocoyl sarcosinate, cocoyl arginate, cocoyl lysinate, cocoyl threoninate, and cocoyl prolinate.

The teachings of the present invention may not only be carried out using biological macromolecules having the exact amino acid or nucleic acid sequences referred to in this application explicitly, for example by name or accession number, or implicitly, but also using variants of such sequences. The term "variant", as used herein, comprises amino acid or nucleic acid sequences, respectively, that are at least 70, 75, 80, 85, 90, 92, 94, 95, 96, 97, 98 or 99 % identical to the reference amino acid or nucleic acid sequence, wherein preferably amino acids other than those essential for the function, for example the catalytic activity of a protein, or the fold or structure of a molecule are deleted, substituted or replaced by insertions or essential amino acids are replaced in a conservative manner to the effect that the biological activity of the reference sequence or a molecule derived therefrom is preserved. The state of the art comprises algorithms that may be used to align two given nucleic acid or amino acid sequences and to calculate the degree of identity, see Arthur Lesk (2008), Thompson *et al.,* 1994, and Katoh *et al.,* 2005. The term "variant" is used synonymously and interchangeably with the term "homologue". Such variants may be prepared by introducing deletions, insertions or substitutions in amino acid or nucleic acid sequences as well as fusions comprising such macromolecules or variants thereof. In one example, the term "variant", with regard to amino acid sequence, comprises, in addition to the above sequence identity, amino acid sequences that comprise one or more conservative amino acid changes with respect to the respective reference or wild type sequence or comprises nucleic acid sequences encoding amino acid sequences that comprise one or more conservative amino acid changes. In one example, the term "variant" of an amino acid sequence or nucleic acid sequence comprises, in addition to the above degree of sequence identity, any active portion and/or fragment of the amino acid sequence or nucleic acid sequence, respectively, or any nucleic acid sequence encoding an active portion and/or fragment of an amino acid sequence. The term "active portion", as used herein, refers to an amino acid sequence or a nucleic acid sequence, which is less than the full length amino acid sequence or codes for less than the full length amino acid sequence, respectively, wherein the amino acid sequence or the amino acid sequence encoded, respectively retains at least some of its essential biological activity. For example an active portion and/or fragment of a protease may be capable of hydrolysing peptide bonds in polypeptides. The phrase "retains at least some of its essential biological activity", as used herein, means that the amino acid sequence in question has a biological activity exceeding and distinct from the background activity and the kinetic parameters characterising said activity, more specifically $k_{cat}$ and $K_M$, are preferably within 3, 2, or 1 order of magnitude of the values displayed by the reference molecule with respect to a specific substrate. Similarly, the term "variant" of a nucleic acid comprises nucleic acids the complementary strand of which hybridises, preferably under stringent conditions, to the reference or wild type nucleic acid. A skilled person would be able to easily determine the amino acid-N-acyl-transferases that will be capable of making proteinogenic amino acids and/or fatty acids. In particular, the variants may include but are not limited to an amino acid-N-acyl-transferase selected from the group of organisms consisting of *Nomascus leucogenys* (Nl, XP_003275392.1), *Saimiri boliviensis* (Sb, XP_003920208.1), *Felis catus* (Fc, XP_003993512.1), *Bos taurus* (Bt, NP_001178259.1), *Mus musculus* (Mm, NP_666047.1) and the like.

Stringency of hybridisation reactions is readily determinable by one ordinary skilled in the art, and generally is an empirical calculation dependent on probe length, washing temperature and salt concentration. In general, longer probes require higher temperatures for proper annealing, while shorter probes need lower temperatures. Hybridisation generally depends on the ability of denatured DNA to reanneal to complementary strands when present in an environment below their melting temperature. The higher the degree of desired homology between the probe and hybridisable sequence, the higher the relative temperature which may be used. As a result it follows that higher relative temperatures would tend to make the reaction conditions more stringent, while lower temperature less so. For additional details and explanation of stringency of hybridisation reactions, see F. M. Ausubel (1995). The person skilled in the art may follow the instructions given in the manual "The DIG System Users Guide for Filter Hybridization", Boehringer Mannheim GmbH, Mannheim, Germany, 1993 and in Liebl *et al.,* 1991 on how to identify DNA sequences by means of hybridisation. In one example, stringent conditions are applied for any hybridisation, i.e. hybridisation occurs only if the probe is 70 % or more identical to the target sequence. Probes having a lower degree of identity with respect to the target sequence may hybridise, but such hybrids are unstable and will be removed in a washing step under stringent conditions, for example by lowering the concentration of salt to 2 x SSC or, optionally and subsequently, to 0,5 x SSC, while the temperature is, in order of increasing preference, approximately 50 °C - 68 °C, approximately 52 °C - 68 °C, approximately 54 °C - 68 °C, approximately 56 °C - 68 °C, approximately 58 °C - 68 °C, approximately 60 °C - 68 °C, approximately 62 °C - 68 °C, approximately 64 °C - 68 °C, approximately 66 °C - 68 °C. In a particularly preferred embodiment, the temperature is approximately 64 °C - 68 °C or approximately 66 °C - 68 °C. It is possible to adjust the concentration of salt to 0.2 x SSC or even 0.1 x SSC. Polynucleotide fragments having a degree of identity with respect to the reference or wild type sequence of at least 70, 80, 90, 91, 92, 93, 94, 95, 96, 97, 98, 99 % may be isolated. The term "homologue" of a nucleic acid sequence, as used herein, refers to any nucleic acid sequence that encodes the same amino acid sequence as the reference nucleic acid sequence, in line with the degeneracy of the genetic code.

The cell according to any aspect of the present invention may comprise a further genetic mutation that increases the expression of at least one transporter protein relative to the wild type cell. This further mutation enables the cell to increase the uptake of at least one fatty acid. In particular, the transporter protein may be AlkL (SEQ ID NO: 5 or 13) and/or FadL (SEQ ID NO: 6). AlkL and/or FadL may function as at least one transporter protein compared to the wild type cell. In one example, the cell may be genetically modified to overexpress both the fadL and the alkL gene.

The cell according to any aspect of the present invention may have reduced capacity of fatty acid degradation by beta-oxidation relative to the wild type cell. In particular, the reduced fatty acid degradation activity compared to the wild type cell, may be a result of decreased expression relative to the wild type cell of at least one enzyme ($E_4$) selected from the group consisting of acyl-CoA dehydrogenase (FadE) ($E_{4a}$), enoyl-CoA hydratase (FadB) ($E_{4b}$), (R)-3-hydroxyacyl-CoA dehydrogenase (FadB) ($E_{4c}$) and 3-ketoacyl-CoA thiolase (FadA) ($E_{4d}$).

The term "having a reduced fatty acid degradation capacity", as used herein, means that the respective cell degrades fatty acids, in particular those taken up from the environment, at a lower rate than a comparable cell or wild type cell having normal fatty acid degradation capacity would under identical conditions. In one example, the fatty acid degradation of such a cell is lower on account of deletion, inhibition or inactivation of at least one gene encoding an enzyme involved in the β-oxidation pathway. In one example, at least one enzyme involved in the β-oxidation pathway has lost, in order of increasing preference, 5, 10, 20, 40, 50, 75, 90 or 99 % activity relative to the activity of the same enzyme under comparable conditions in the respective wild type microorganism. The person skilled in the art may be familiar with various techniques that may be used to delete a gene encoding an enzyme or reduce the activity of such an enzyme in a cell, for example by exposition of cells to radioactivity followed by accumulation or screening of the resulting mutants, site-directed introduction of point mutations or knock out of a chromosomally integrated gene encoding for an active enzyme, as described in Sambrook/Fritsch/Maniatis (1989). In addition, the transcriptional repressor FadR may be over expressed to the effect that expression of enzymes involved in the β-oxidation pathway is repressed (Fujita, Y., et al, 2007). The phrase "deletion of a gene", as used herein, means that the nucleic acid sequence encoding said gene is modified such that the expression of active polypeptide encoded by said gene is reduced. For example, the gene may be deleted by removing in-frame a part of the sequence comprising the sequence encoding for the catalytic active centre of the polypeptide. Alternatively, the ribosome binding site may be altered such that the ribosomes no longer translate the corresponding RNA. It would be within the routine skills of the person skilled in the art to measure the activity of enzymes expressed by living cells using standard essays as described in enzymology text books, for example Cornish-Bowden, 1995.

Degradation of fatty acids is accomplished by a sequence of enzymatically catalysed reactions. First of all, fatty acids are taken up and translocated across the cell membrane *via* a transport/acyl-activation mechanism involving at least one outer membrane protein and one inner membrane-associated protein which has fatty acid-CoA ligase activity, referred to in the case of *E. coli* as FadL and FadD / FadK, respectively. Inside the cell, the fatty acid to be degraded is subjected to enzymes catalysing other reactions of the β-oxidation pathway. The first intracellular step involves the conversion of acyl-CoA to enoyl-CoA through acyl-CoA dehydrogenase, the latter referred to as FadE in the case of *E. coli.* The activity of an acyl-CoA dehydrogenase may be assayed as described in the state of art, for example by monitoring the concentration of NADH spectrophotometrically at 340 nm in 100 mM MOPS, pH 7.4, 0.2 mM Enoyl-CoA, 0.4 mM NADH. The resulting enoyl-CoA is converted to 3-ketoacyl-CoA *via* 3-hydroxyacyl-CoA through hydration and oxidation, catalysed by enoyl-CoA hydratase/(R)-3-hydroxyacyl-CoA dehydrogenase, referred to as FadB and FadJ in *E. coli.* Enoyl-CoA hydratase/3-hydroxyacyl-CoA dehydrogenase activity, more specifically formation of the product NADH may be assayed spectrophotometrically as described in the state of the art, for example as outlined for FadE. Finally, 3-ketoacyl-CoA thiolase, FadA and FadI in *E. coli,* catalyses the cleavage of 3-ketoacyl-CoA, to give acetyl-CoA and the input acyl-CoA shortened by two carbon atoms. The activity of ketoacyl-CoA thiolase may be assayed as described in the state of the art, for example in Antonenkov, V., et al, 1997.

The phrase "a cell having a reduced fatty acid degradation capacity", as used herein, refers to a cell having a reduced capability of taking up and/or degrading fatty acids, particularly those having at least eight carbon chains. The fatty acid degradation capacity of a cell may be reduced in various ways. In particular, the cell according to any aspect of the present invention has, compared to its wild type, a reduced activity of an enzyme involved in the β-oxidation pathway. The term "enzyme involved in the β-oxidation pathway", as used herein, refers to an enzyme that interacts directly with a fatty acid or a derivative thereof formed as part of the degradation of the fatty acid via the β-oxidation pathway. The β-oxidation pathway comprises a sequence of reactions effecting the conversion of a fatty acid to acetyl-CoA and the CoA ester of the shortened fatty acid. The enzyme involved in the β-oxidation pathway may by recognizing the fatty acid or derivative thereof as a substrate, converts it to a metabolite formed as a part of the β-oxidation pathway. For example, the acyl-CoA dehydrogenase is an enzyme involved in the β-oxidation pathway as it interacts with fatty acid-CoA and converts fatty acid-CoA ester to enoyl-CoA, which is a metabolite formed as part of the β-oxidation. In another example, the term "enzyme involved in the β-oxidation pathway", as used herein, comprises any polypeptide from the group comprising acyl-CoA dehydrogenase, enoyl-CoA hydratase, 3-hydroxyacyl-CoA dehydrogenase and 3-keto-acyl-CoA thiolase. The acyl-CoA synthetase may catalyse the conversion of a fatty acid to the CoA ester of a fatty acid, *i.e.* a molecule, wherein the functional group -OH of the carboxy group is replaced with - S-CoA and introducing the fatty acid into the β-oxidation pathway. For example, the polypeptides FadD and FadK in *E. coli* (accession number: BAA15609.1 and NP_416216.4, respectively) are acyl-CoA synthetases. In one example, the term "acyl-CoA dehydrogenase", as used herein, may be a polypeptide capable of catalysing the conversion of an acyl-CoA to enoyl-CoA, as part of the β-oxidation pathway. For example, the polypeptide FadE in *E. coli* (accession number: BAA77891.2) may be an acyl-CoA dehydrogenase. The term "enoyl-CoA hydratase", as used herein, also referred to as 3-hydroxyacyl-CoA dehydrogenase, refers to a polypeptide capable of catalysing the conversion of enoyl-CoA to 3-ketoacyl-CoA through hydration and oxidation, as part of the β-oxidation pathway. For example, the polypeptides FadB and FadJ in *E. coli* (accession numbers: BAE77457.1 and P77399.1, respectively) are enoyl-CoA hydratases. The term "ketoacyl-CoA thiolase", as used herein, may refer to a polypeptide capable of catalysing the cleaving of 3-ketoacyl-CoA, resulting in an acyl-CoA shortened by two carbon atoms and acetyl-CoA, as the final step of the β-oxidation pathway. For example, the polypeptides FadA and FadI in *E. coli* (accession number: YP_491599.1and P76503.1, respectively) are ketoacyl-CoA thiolases.

Any of the enzymes used according to any aspect of the present invention, may be an isolated enzyme. In particular, the enzymes used according to any aspect of the present invention may be used in an active state and in the presence of all cofactors, substrates, auxiliary and/or activating polypeptides or factors essential for its activity. The term "isolated", as used herein, means that the enzyme of interest is enriched compared to the cell in which it occurs naturally. The enzyme may be enriched by SDS polyacrylamide electrophoresis and/or activity assays. For example, the enzyme of interest may constitute more than 5, 10, 20, 50, 75, 80, 85, 90, 95 or 99 percent of all the polypeptides present in the preparation as judged by visual inspection of a polyacrylamide gel following staining with Coomassie blue dye.

The enzyme used according to any aspect of the present invention may be recombinant. The term "recombinant" as used herein, refers to a molecule or is encoded by such a molecule, particularly a polypeptide or nucleic acid that, as such, does not occur naturally but is the result of genetic engineering or refers to a cell that comprises a recombinant molecule. For example, a nucleic acid molecule is recombinant if it comprises a promoter functionally linked to a sequence encoding a catalytically active polypeptide and the promoter has been engineered such that the catalytically active polypeptide is overexpressed relative to the level of the polypeptide in the corresponding wild type cell that comprises the original unaltered nucleic acid molecule.

Whether or not a nucleic acid molecule, polypeptide, more specifically an enzyme used according to any aspect of the present invention, is recombinant or not has not necessarily implications for the level of its expression. However, in one example one or more recombinant nucleic acid molecules, polypeptides or enzymes used according to any aspect of the present invention may be overexpressed. The term "overexpressed", as used herein, means that the respective polypeptide encoded or expressed is expressed at a level higher or at higher activity than would normally be found in the cell under identical conditions in the absence of genetic modifications carried out to increase the expression, for example in the respective wild type cell. The person skilled in the art is familiar with numerous ways to bring about overexpression. For example, the nucleic acid molecule to be overexpressed or encoding the polypeptide or enzyme to be overexpressed may be placed under the control of a strong inducible promoter such as the lac promoter. The state of the art describes standard plasmids that may be used for this purpose, for example the pET system of vectors exemplified by pET-3a (commercially available from Novagen). Whether or not a nucleic acid or polypeptide is overexpressed may be determined by way of quantitative PCR reaction in the case of a nucleic acid molecule, SDS polyacrylamide electrophoreses, Western blotting or comparative activity assays in the case of a polypeptide. Genetic modifications may be directed to transcriptional, translational, and/or post-translational modifications that result in a change of enzyme activity and/or selectivity under selected and/or identified culture conditions. Thus, in various examples of the present invention, to function more efficiently, a microorganism may comprise one or more gene deletions. Gene deletions may be accomplished by mutational gene deletion approaches, and/or starting with a mutant strain having reduced or no expression of one or more of these enzymes, and/or other methods known to those skilled in the art.

The cell according to any aspect of the present invention may refer to a wide range of microbial cells. In particular, the cell may be selected from the group consisting of *Pseudomonas, Corynebacterium, Bacillus* and *Escherichia.* In one example, the cell may be *Escherichia coli.* In another example, the cell may be a lower eukaryote, such as a fungus from the group comprising *Saccharomyces, Candida, Pichia, Schizosaccharomyces* and *Yarrowia,* particularly, *Saccharomyces cerevisiae.* The microorganism may be an isolated cell, in other words a pure culture of a single strain, or may comprise a mixture of at least two strains. Biotechnologically relevant cells are commercially available, for example from the American Type Culture Collection (ATCC) or the German Collection of Microorganisms and Cell Cultures (DSMZ). Particles for keeping and modifying cells are available from the prior art, for example Sambrook/Fritsch/Maniatis (1989).

In one example, the cell according to any aspect of the present invention may be capable of producing sufficient fatty acids from the natural oil used as a substrate that no further fatty acid has to be added as a substrate. In another example, the amount of fatty acids and/ amino acids used as substrate may be supplemented to increase the production of acyl amino acids.

In this example, the cell may thus be genetically modified to:

- increase the expression relative to the wild type cell of an amino acid-N-acyl-transferase ($E_2$) and an acyl-CoA synthetase ($E_3$),
- increase the expression relative to the wild type cell of a lipase ($E_1$), and
- increase the expression relative to the wild type cell of an enzyme associated to production of proteinogenic amino acids and/or fatty acids from at least one carbohydrate.

The term "proteinogenic amino acid", as used herein, refers to an amino acid selected from the group comprising alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine and valine. Proteinogenic amino acids and fatty acids are synthesized as part of the primary metabolism of many wild type cells in reactions and using enzymes that have been described in detail in biochemistry textbooks, for example Jeremy M Berg, et al., 2002. In particular, the proteinogenic amino acid may be selected from the group consisting of glycine, glutamine, glutamate, asparagine and alanine. More in particular, the proteinogenic amino acid may be glycine.

The cell according to any aspect of the present invention may comprise at least one genetic mutation that enables the cell to produce at least one fatty acid according to any method known in the art. In one example, the genetic mutation may enable the cell to produce at least one fatty acid by means of a malonyl-CoA dependent and malonyl-ACP independent fatty acyl-CoA metabolic pathway. This is further described in US20140051136.

In another example, the cell according to any aspect of the present invention may be capable of producing its own amino acid. Different enzymes may be selected based on the amino acid to be produced by the cell. In one example, the amino acid to be produced by the cell may be glycine. In particular, the cell according to any aspect of the present invention may comprise a fourth genetic mutation to increase the expression relative to the wild type cell of at least one enzyme ($E_6$) that may be capable of enabling the cell to produce glycine. More in particular, the enzyme ($E_6$) may be at least one enzyme from the glycine cleavage system also referred to as the glycine synthase. Even more in particular, the enzyme may be selected from the group consisting of T-protein (EC 2.1.2.10) ($E_{6a}$) an aminomethyltransferase, P-protein (EC 1.4.4.2) ($E_{6b}$) a glycine dehydrogenase, L-protein (EC 1.8.1.4) ($E_{6c}$) a dihydrolipoyl dehydrogenase and H-protein ($E_{6d}$). The cell according to any aspect of the present invention may have an increased expression of any one of the enzymes ($E_{6a}$-$E_{6d}$) relative to the wild type cell to result in the production of glycine in the cell according to any aspect of the present invention. In another example, the enzyme ($E_6$) may at least be one serine hydroxymethyltransferase (*GlyA*) ($E_{6e}$) (EC:2.1.2.1) where serine may be used as an intermediate. The cell according to any aspect of the present invention may have an increased expression of $E_{6e}$ relative to the wild type cell to result in the production of glycine in the cell according to any aspect of the present invention. In another example, the enzyme ($E_6$) may be a threonine adolase (*LtaE*) ($E_{6f}$) (EC: 4.1.2.5) or threonine dehydrogenase (*Tdh*) ($E_{6g}$) (EC: 1.1.1.103) and glycine C-acyltransferase ($E_{6h}$) also known as 2-Amino-3-Ketobutyrate CoA-Ligase (Kbl) (EC: 2.3.1.29), The cell according to any aspect of the present invention may have an increased expression of $E_{6f}$ or $E_{6g}$ and $E_{6h}$ relative to the wild type cell to result in the production of glycine in the cell according to any aspect of the present invention. In another example, the cell according to any aspect of the present invention may have an increased expression of $E_{6f}$ and $E_{6g}$ in combination with Allothreonine dehydrogenase (L-allo-threonine dehydrogenase, (YdfG)).

In another example, the forth genetic mutation in the cell according to any aspect of the present invention may comprise a combination of enzymes ($E_{6a}$ to $E_{6h}$) that allow the cell to produce glycine.

The forth mutation in the cell according to any aspect of the present invention may be in any enzyme that results in the cell producing a target amino acid. In one example, the cell may be capable of producing an amino acid and a fatty acid that may provide the substrates for acyl amino acid production.

In another example, the cell according to any aspect of the present invention may comprise a further mutation in at least one enzyme selected from the group consisting of:

(i) An enzyme ($E_7$) capable of uptake of glutamate; and
(ii) An enzyme ($E_8$) capable of interconverting acyl-CoAs and acyl-ACPs.

In particular, $E_7$ may be a glutamate-translocating ABC transporter or permease; and $E_8$ may be an acyl-CoA:ACP transacylase.

According to another aspect of the present invention, there is provided a method of producing at least one acyl amino acid in an aqueous medium comprising at least one lipase ($E_1$) and at least one glyceride and amino acid, wherein the method comprises a step of:

- contacting at least one cell comprising a genetic mutation that increases the expression relative to a wild type cell of an amino acid-N-acyl-transferase ($E_2$) and an acyl-CoA synthetase ($E_3$) with the aqueous medium, and

wherein the lipase ($E_1$) is capable of hydrolysing the glyceride to at least one fatty acid and the cell is capable of converting the fatty acid and amino acid to the acyl amino acid, and
wherein the cell has a reduced fatty acid degradation capacity.

According to yet another aspect of the present invention, there is provided a method of producing at least one acyl amino acid, wherein the method comprises the step of contacting at least one cell according to any aspect of the present invention to an aqueous medium comprising at least one glyceride and amino acid

According to any aspect of the present invention, the lipase ($E_1$) may be provided externally (i.e. in the aqueous medium) where the cell is grown for the production of acyl amino acids or the lipase ($E_1$) may be part of the DNA of the cell grown in the aqueous medium to produce acyl amino acids. In whichever scenario the lipase ($E_1$) is introduced into the method according to any aspect of the present invention, the lipase may be capable of hydrolysing the glyceride in the aqueous medium to produce at least one fatty acid and glycerol.

In one example, where lipase ($E_1$) is part of the aqueous medium, the concentration of lipase may vary depending on the amount of natural oils present in the medium. The added amount of enzyme, reaction time, or the like must be set to obtain a balance between the obtained oil product yield and efficiency of concentration of the target fatty acid. In one example, the utilised amount of lipase relative to 1 g of triglyceride may be from 10 to 2,000 units, and particularly it may be from 200 to 700 units. Here, 1 unit is the amount of enzyme that releases 1 $\mu$mol of fatty acid in 1 minute. The hydrolysis reaction using lipase requires that the reaction be performed in the presence of a sufficient amount of water for expression of the hydrolysis activity of lipase. Relative to the triglyceride, the amount of water present is from 10 to 200 percent by weight, and particularly may be from 50 to 150 percent by weight.

Optimum temperature of the enzyme reaction is known to depend on the enzyme, and reactions are performed within the temperature range. Although lipase reacts within the temperature range thereof, viscosity of the target oil of the lipase reaction increases at low temperature, and the effectiveness of stirring the oil and enzyme-containing water worsens. Thus, the reaction is normally performed at from 30 to 40°C. For example, when *Candida cylindracea*-derived lipase is used for concentration of polyunsaturated fatty acid, the reaction temperature may be room temperature or as high as 37°C.

The term "contacting", as used herein, means bringing about direct contact between the amino acid, the fatty acid and/or the cell according to any aspect of the present invention in an aqueous solution. For example, the cell, the amino acid and the fatty acid may not be in different compartments separated by a barrier such as an inorganic membrane. If the amino acid or fatty acid is soluble and may be taken up by the cell or can diffuse across biological membranes, it may simply be added to the cell according to any aspect of the present invention in an aqueous solution. In case it is insufficiently soluble, it may be solved in a suitable organic solvent prior to addition to the aqueous solution. The person skilled in the art is able to prepare aqueous solutions of amino acids or fatty acids having insufficient solubility by adding suitable organic and/or polar solvents. Such solvents may be provided in the form of an organic phase comprising liquid organic solvent. In one example, the organic solvent or phase may be considered liquid when liquid at 25 °C and standard atmospheric pressure. In another example, a fatty acid may be provided in the form of a fatty acid ester such as the respective methyl or ethyl ester. In another example, the compounds and catalysts may be contacted *in vitro,* i.e. in a more or less enriched or even purified state, or may be contacted *in situ,* i.e. they are made as part of the metabolism of the cell and subsequently react inside the cell.

The term "an aqueous solution" is used interchangeably with the term 'aqueous medium" and refers to any solution comprising water, mainly water as solvent that may be used to keep the cell according to any aspect of the present invention, at least temporarily, in a metabolically active and/or viable state and comprises, if such is necessary, any additional substrates. The person skilled in the art is familiar with the preparation of numerous aqueous solutions, usually referred to as media that may be used to keep inventive cells, for example LB medium in the case of *E. coli.* It is advantageous to use as an aqueous solution a minimal medium, *i.e*. a medium of reasonably simple composition that comprises only the minimal set of salts and nutrients indispensable for keeping the cell in a metabolically active and/or viable state, by contrast to complex mediums, to avoid dispensable contamination of the products with unwanted side products. For example, M9 medium may be used as a minimal medium.

The method according to any aspect of the present invention may be used to convert both saturated and unsaturated fatty acids from the naturally occurring oils, to acyl amino acids. In case the end product sought-after is to comprise a higher yield of saturated acyl residues than is present, it may be possible to complement the method according to any aspect of the present invention by hydrogenating the acyl residues of the acyl amino acids. The resultant composition would thus comprise a mixture of saturated acyl residues. The hydrogenation may be carried out according to various state of the art processes, for example those described in US5734070. Briefly, the compound to be hydrogenated may be incubated at 100 °C in the presence of hydrogen and a suitable catalyst, for example a nickel catalyst on silicon oxide as a support.

The fatty acids and/or amino acids that are to be converted to acyl amino acids may be added in the form of naturally occurring oils and an amino acid in the aqueous medium according to any aspect of the present invention. In another example, to supplement the substrate of fatty acid (from the naturally occurring oils), the cell that produces the acyl amino acids is capable of producing the fatty acids from which the acyl amino acids are produced. In particular, the cells may be genetically modified to be able to produce fatty acids and/or amino acids. In one example, the genetic modification may be to decrease a specific enzymatic activity and this may be done by a gene disruption or a genetic modification. The genetic modification may also increase a specific enzymatic activity. In particular, the genetic modification may increase microbial synthesis of a selected fatty acid or fatty acid derived chemical product above a rate of a control or wild type cell. This control or wild type cell may lack this genetic modification to produce a selected chemical product.

According to a further aspect of the present invention, there is provided a use of at least one cell according to any aspect of the present invention for the production of at least one acyl amino acid. In particular, the acyl amino acid may be fatty acid-acyl glycinate. More in particular, the fatty acid acyl glycinate may be selected from the group consisting of lauroyl glycinate, myristoyl glycinate and palmitoyl glycinate

In particular, the method according to any aspect of the present invention is carried out within the cell according to any aspect of the present invention.

## BRIEF DESCRIPTION OF THE FIGURES

No Figures

## EXAMPLES

The foregoing describes preferred embodiments, which, as will be understood by those skilled in the art, may be subject to variations or modifications in design, construction or operation without departing from the scope of the claims. These variations, for instance, are intended to be covered by the scope of the claims.

### Example 1

*Production of acyl amino acids from coconut oil*

The strain E.coli W3110 ΔfadE pCDF{Ptac}[hGLYAT2(co_Ec) / fadD_Ec] {Plavuv5} [alkLmod1] was fermented in a fed-batch fermentation to study the ability of linking fatty acids from coconut oil and glycine to fatty glycinates using an external lipase from *Thermomyces lanuginosus.*

The strain was used for studying their ability to produce fatty acid amino acid adducts with glycine and coconut oil. For this purpose, the strain was cultured both in shake flask and in fed-batch fermentation. The fermentation was carried out in a parallel fermentation system from DASGIP with 8 bioreactors.

The fermentation was performed using 1 l reactors equipped with overhead stirrers and impeller blades. pH and $pO_2$ were measured online for process monitoring. OTR/CTR measurements served for estimating the metabolic activity and cell fitness, inter alia.

The pH electrodes were calibrated by means of a two-point calibration using standard solutions of pH 4.0 and pH 7.0, as specified in DASGIP's technical instructions. The reactors were provided with the necessary sensors and connections as specified in the technical instructions, and the agitator shaft was fitted. The reactors were then charged with 300 mL water and autoclaved for 20 min at 121 °C to ensure sterility. The $pO_2$ electrodes were connected to the measuring amplifiers and polarized overnight (for at least 6 h). Thereafter, the water was removed under a clean bench and replaced by M9 medium (pH 7.4) composed of $KH_2PO_4$ 3.0 g/l, $Na_2HPO_4$ 6.79 g/l, NaCl 0.5 g/l, $NH_4Cl$ 2.0 g/l, 2 mL of a sterile 1 M $MgSO_4*7H_2O$ solution and 1 mL/l of a filter-sterilized trace element stock solution (composed of HCl (37%) 36.50 g/L, $MnCl_2*4H_2O$ 1.91 g/L, $ZnSO_4*7H_2O$ 1.87 g/L, ethylenediaminetetraacetic acid dihydrate 0.84 g/L, $H_3BO_3$ 0.30 g/L, $Na_2MoO_4*2H_2O$ 0.25 g/L, $CaCl_2*2H_2O$ 4.70 g/L, $FeSO_4*7H_2O$ 17.80 g/L, $CuCl_2*2H_2O$ 0.15 g/L) with 15 g/L glucose as the carbon source (added by metering in 30 mL/L of a sterile feed solution composed of 500 g/L glucose, 1.3 % (w/v) $MgSO_4*7H_2O$) supplemented with 100 mg/L spectinomycin and 0.5 mL/L Delamex.

Thereafter, the $pO_2$ electrodes were calibrated to 100 % with a one-point calibration (stirrer: 400 rpm/aeration: 10 sl/h air), and the feed, correction agent and induction agent lines were cleaned by "cleaning in place" as specified in the technical instructions. To this end, the tubes were rinsed first with 70 % ethanol, then with 1 M NaOH, then with sterile fully-demineralized water and, finally, filled with the respective media.

Using the *E. coli* strain W3110 ΔfadE pCDF{Ptac}[hGLYAT2(co_Ec) / fadD_Ec] {Plavuv5} [alkLmod1], LB medium (10 mL in a 100-mL baffle flask) supplemented with 100 mg/l spectinomycin was inoculated 100 μL of a cryo-culture and the culture was grown at 37 °C and 200 rpm for approximately 14 h. Thereafter, this culture was used for a second preculture stage with an initial OD of 0.2 in 50 mL of M9 medium, composed of $KH_2PO_4$ 3.0 g/l, $Na_2HPO_4$ 6.79 g/l, NaCl 0.5 g/l, $NH_4Cl$ 2.0 g/l, 2 mL of a sterile 1 M $MgSO_4$*$7H_2O$ solution and 1 mL/l of a filter-sterilized trace element stock solution (composed of HCl (37 %) 36.50 g/L, $MnCl_2$*$4H_2O$ 1.91 g/L, $ZnSO_4$*$7H_2O$ 1.87 g/L, ethylenediaminetetraacetic acid dihydrate 0.84 g/l, $H_3BO_3$ 0.30 g/l, $Na_2MoO_4$*$2H_2O$ 0.25 g/L, $CaCl_2$*$2H_2O$ 4.70 g/L, $FeSO_4$*$7H_2O$ 17.80 g/L, $CuCl_2$*$2H_2O$ 0.15 g/L) supplemented with 15 g/L glucose as carbon source (added by metering in 30 mL/L of a sterile feed solution composed of 500 g/L glucose) together with the above-described antibiotics was transferred into a 500-mL baffle flask and incubated for 8-12 h at 37 °C/200 rpm.

To inoculate the reactors with an optical density of 0.1, the $OD_{600}$ of the second preculture stage was measured and the amount of culture required for the inoculation was calculated. The required amount of culture was placed into the heated and aerated reactor with the aid of a 5-mL syringe through a septum.

The standard program shown in Table 2a-c was used:

Table 2. Standard program for use of heated and aerated reactor

| a) | | | |
|---|---|---|---|
| DO controller | | pH controller | |
| Preset | 0% | Preset | 0 mL/h |
| P | 0.1 | P | 5 |
| Ti | 300 s | Ti | 200 s |
| Min | 0% | Min | 0 mL/h |
| Max | 100 % | Max | 40 mL/h |

| b) | | | | | | | | |
|---|---|---|---|---|---|---|---|---|
| N (Rotation) | From | To | XO2 (gas mixture) | from | to | F (gas flow) | from | to |
| Growth and biotrans-formation | 0% | 30 % | Growth and biotrans-formation | 0% | 100 % | Growth and biotrans-formation | 15% | 80 % |
| | 400 rpm | 1500 rpm | | 21 % | 21 % | | 6 sl/h | 72 sl/h |

| c) | |
|---|---|
| Script | |
| Trigger fires | 31 % DO (1/60h) |
| Temperature | 37 °C |
| Induction IPTG | 2 h after the feed start |
| Feed trigger | 50 % DO |
| Feed rate | 3 [mL/h] |

The pH was adjusted unilaterally to pH 7.0 with 12.5 % strength ammonia solution. During the growth phase and the biotransformation, the dissolved oxygen ($pO_2$ or DO) in the culture was adjusted to at least 30 % via the stirrer speed and the aeration rate. After the inoculation, the DO dropped from 100 % to these 30 %, where it was maintained stably for the remainder of the fermentation.

The fermentation was carried out as a fed batch, the feed start as the beginning of the feed phase with 5 g/l*h glucose feed, composed of 500 g/l glucose, 1.3 % (w/v) $MgSO_4$*$7H_2O$, being triggered via the DO peak which indicates the end of the batch phase. From the feed start onwards, the temperature was reduced from 37 °C to 30 °C. 2 h after the feed start, the expression was induced with 1 mM IPTG.

In the experimental setting the biotransformation starts 2 h after induction 100 g/l glycine in demineralized water (100 mL/L) and 5 g coconut oil (heated) were fed rather than glucose. Start of the reaction was the addition of the coconut oil. At this time 20 μL of a lipase solution (approx.. 2000 U) was also added to the medium.

To quantify lauroyl, myristoyl and palmitoyl glycinate in fermentation samples, samples were taken 23 h and 42 h after the start of the fermentation. These samples were analysed and the results are shown in Table 3.

It has been possible to demonstrate that the strain *E.coli* W3110 ΔfadE pCDF{Ptac}[hGLYAT2(co_Ec)_fadD_Ec]{Plavuv5} [alkLmod1] using an external lipase is capable of linking fatty acids after cleaving of the coconut oil and glycine producing fatty acid glycinates.

| Fermentation time [h] | $C_{Lauroyl\ glycinate}$ [g/L] | $C_{Myristoyl\ glycinate}$ [g/L] | $C_{Palmitoyl\ glycinate}$ [g/L] | $C_{Glycine}$ [g/L] | $C_{Octanoic\ acid}$ [g/L] | $C_{Decanoic\ acid}$ [g/L] | $C_{Lauric\ acid}$ [g/L] | $C_{Myristic\ acid}$ [g/L] | $C_{Palmitoleic\ acid}$ [g/L] | $C_{Palmitic\ acid}$ [g/L] | $C_{Oleic\ acid}$ [g/L] | $C_{Stearic\ acid}$ [g/L] |
|---|---|---|---|---|---|---|---|---|---|---|---|---|
| 25 | 1.79 | 0.86 | 0.41 | 7.6 | < 0.1 | < 0.1 | < 0.1 | < 0.1 | < 0.1 | < 0.1 | < 0.1 | < 0.1 |
| 29 | 2.16 | 0.92 | 0.47 | 4.7 | < 0.1 | < 0.1 | < 0.1 | < 0.1 | < 0.1 | < 0.1 | < 0.1 | < 0.1 |
| 40 | 2.62 | 0.85 | 0.43 | <0.1 | < 0.1 | < 0.1 | < 0.1 | < 0.1 | < 0.1 | < 0.1 | < 0.1 | < 0.1 |
| 45 | 1.96 | 0.62 | 0.32 | n.d. | < 0.1 | < 0.1 | < 0.1 | < 0.1 | < 0.1 | < 0.1 | < 0.1 | < 0.1 |

Table 3: Quantification of fatty acid glycinates after 25, 29, 40 and 45 h fermentation time.

**Example 2**

*Generation of a vector for expression of the Lipase gene of Thermomyces lanuginosus in Escherichia coli W3110 ΔfadE*

To generate a vector for the expression of the Lipase gene of *Thermomyces lanuginosus* (lipTI) (Seq-ID NO:. 4) a synthetic gene-fusion of the target gene *lipTI* with an *E. coli* secretion signal *ompA* was used (Sletta et al. 2007, Movva et al. 1980). The synthetic, *E. coli* codon-optimized fusion was amplified using the oligonucleotides ompA_fw (SEQ ID NO:7) and lipTI_rev (SEQ ID NO:8) to generate overlaps to the target vector pQE80L-kan (SEQ ID NO:9) for cloning purposes. A PCR fragment of the expected size could be amplified (985 bp, (SEQ ID NO:10), was separated via agarose gel electrophoresis and isolated with *QiaQuick Gel extraction Kit* (Qiagen, Hilden). The purified PCR fragment was assembled with the EcoRI/BamHI cut vector pQE80L-kan using the Gibson Assembly®-Kit ((New England Biolabs, Frankfurt) and transformed into chemically competent *E. coli* DH5α cells (New England Biolabs, Frankfurt). Procedure of PCR purification, in-vitro cloning and transformation were carried out according to manufacturer's manual. The correct insertion of the target genes was checked by restriction analysis and the authenticity of the introduced genes was verified by DNA sequencing. The resulting vector was named pQE80L-kan{PT5}[ompA-lipTI(co_Ec)] (SEQ ID NO:11).

The plasmids pCDF{Ptac}[hGLYAT2(co_Ec)/fadD_Ec] {Plavuv5} [alkLmod1] and pQE80L-kan{PT5}[ompA-lipTI(co_Ec)] were co-transformed into the strain *E.coli* W3110 ΔfadE by means of electroporation and plated onto LB-agar plates supplemented with spectinomycin (50-100 μg/mL) and kanamycin (50 μg/mL). Transformants were checked for the presence of the correct plasmids by plasmid preparation and analytic restriction analysis. The resulting strain was named *E. coli* W3110 *ΔfadE* pCDF{Ptac}[hGLYAT2(co_Ec) / fadD_Ec] {Plavuv5} [alkLmod1] and pQE80L-kan{PT5} [ompA-LipTI(co_Ec)]. The sequence of the fusion-protein ompA-LipTI is listed as SEQ ID NO:12.

**REFERENCES**

1. Antonenkov, V., D. Van Veldhoven, P., P., Waelkens, E., and Mannaerts, G.P. (1997) J. Biol. Chem. 1997, 272:26023-26031
2. Arthur Lesk (2008), Introduction to bioinformatics, 3rd edition,
3. F. M. Ausubel (1995), Current Protocols in Molecular Biology. John Wiley & Sons, Inc.
4. Barker HA, (1959) JBiol Chem. Feb;234(2):320-8.
5. Barker HA (1967) Arch. Mikrobiol. 59, 4-12
6. Blair AH, Barker HA (1966) J. Biol. Chem. 241, 400-408
7. Badenhorst CP, Drug Metab Dispos. 2012 Feb;40(2):346-52. doi: 10.1124/dmd.111.041657. Epub 2011 Nov 9.
8. Buckel W, Dorn U and Semmler R (1981) Eur. J. Biochem. 118, 315-321

9. Chen HP and Marsh ENG (1997) Biochemistry 36, 14939-14945
10. Cornish-Bowden (1995), Fundamentals of Enzyme Kinetics, Portland Press Limited, 1995
11. Duff SM, (2012) Arch. Biochem. Biophys. 528, 90-101
12. Esser D, (2013) Extremophiles, 17:205-216
13. Feng Y, (2002) Biochemistry 41. Oct 22;41(42):12883-90
14. Fujita, Y., Matsuoka, H., and Hirooka, K. (2007) Mol. Microbiology 66(4), 829-839
15. Härtel U, (1993) Archives of Microbiology. Volume 159, Issue 2, pp 174-181
16. Hawkins AB, (2014) Appl Environ Microbiol. Apr;80(8):2536-45. doi: 10.1128/AEM.04146-13. Epub 2014 Feb 14
17. Jeffery D, (1988) Insect Biochemistry. Volume 18, Issue 4, 1988, Pages 347-349
18. Jeremy M Berg, John L Tymoczko, and Lubert Stryer, Biochemistry, 5th edition, W. H. Freeman, 2002.
19. Kalliri E, (2008) J. Bacteriol. 190, 3793-3798
20. Kang, Y., (2010), PLOS ONE 5 (10), e13557
21. Karmen (1955) J Clin Invest. Jan;34(1):131-3
22. Katoh et al., Genome Information, 16(1), 22-33, 2005.
23. Liebl W., International Journal of Systematic Bacteriology 41: 255-260 (1991)
24. Liu W, (2005) Biochemistry. Mar 1 ;44(8):2982-92
25. Mavrides C (1975) J. Biol. Chem. 250, 4128-4133
26. Moskowitz GJ (1969) Biochemistry. 8(7):2748-55
27. Movva NR, Nakamura K, Inouye M.(1980).. J Biol Chem. Jan 10;255(1):27-9.
28. Parthasarathy A, (2011) Biochemistry. May 3;50(17):3540-50. Epub 2011 Apr 5
29. Sambrook/Fritsch/Maniatis (1989): Molecular cloning -A Laboratory Manual, Cold Spring Harbour Press, 2nd edition, Fuchs/Schlegel (2007), Allgemeine Mikrobiologie, 2008, Georg Thieme Verlag
30. Sletta H, Tødervik A, Hakvåg S, Aune TE, Nedal A, Aune R, Evensen G, Valla S, Ellingsen TE, Brautaset T. (2007). Appl Environ Microbiol. Feb;73(3):906-12 Epub 2006 Dec 1.
31. Söhling B (1993) EurJ Biochem. Feb 15;212(1):121-7
32. Taylor RC, (2010) Microbiology 156, 1975-1982
33. Thompson et al., Nucleic Acids Research 22, 4637-4680, 1994,
34. Waluk, D. (2010), FASEB J. 24, 2795-2803.
35. Wang CC, (1969) J. Biol. Chem. 244, 2516-2526
36. Wiesenborn DP, (1988) Appl. Environ. Microbiol. 54, 2717-2722
37. Yamashita H, (2006) Biochim. Biophys. Acta 1761, 17-23
38. Yu K, (2011) Enzyme Microb Technol. Aug 10;49(3):272-6. doi: 10.1016/j.enzmictec.2011.06.007. Epub2011 Jun 12
39. US5734070, US20140051136

**Claims**

1. A microbial cell for producing at least one acyl amino acid, wherein the cell is genetically modified to comprise:

- a first genetic mutation that increases the expression relative to a wild type cell of at least one lipase (EC 3.1.1) ($E_1$) capable of hydrolysing at least one glyceride to at least one fatty acid; and
- a second and a third genetic mutation that increases the expression relative to a wild type cell of:

(i) an amino acid-N-acyl-transferase (EC 2.3.2) ($E_2$), and
(ii) an acyl-CoA synthetase (EC 6.2.1.1) ($E_3$) respectively

that enables the cell to convert the fatty acid to at least one acyl amino acid and wherein the cell has a reduced fatty acid degradation capacity.

2. The cell according to claim 1, wherein the lipase ($E_1$) is a lipase derived from any one of the microorganisms selected from the group consisting of *Candida cylindracea, Burkholderia cepacia, Pseudomonas fluorescens, Thermomyces lanuginosus,* and *Rhizomucor miehei.*

3. The cell according to either claim 1 or 2, wherein

- $E_1$ comprises SEQ ID NO: 4 or a variant thereof;
- $E_2$ comprises SEQ ID NO: 1 or a variant thereof; and/or
- $E_3$ comprises SEQ ID NO: 2 or a variant thereof.

4. The cell according to any one of the preceding claims, wherein the fatty acid degradation capacity is reduced owing to a decrease in activity, compared to the wild type cell, of at least one enzyme ($E_4$) selected from the group consisting of acyl-CoA dehydrogenase ($E_{4a}$), 2,4-dienoyl-CoA reductase ($E_{4b}$), enoyl-CoA hydratase ($E_{4c}$) and 3-ketoacyl-CoA thiolase ($E_{4d}$).

5. The cell according to any one of the preceding claims, wherein the amino acid-N-acyl-transferase ($E_2$) is glycine-N-acyl-transferase.

6. The cell according to any one of the preceding claims, wherein the cell is capable of making proteinogenic amino acids and/or fatty acids.

7. The cell according to any of the preceding claims, wherein the acyl amino acid is a N-acyl glycinate or N-acyl glutamate.

8. The cell according to any one of the preceding claims, wherein the cell has a further genetic mutation that increases the expression of at least one transporter protein relative to the wild type cell that enables the cell to increase the uptake of at least one fatty acid.

9. The cell according to claim 8, wherein the transporter protein is selected from the group consisting of FadL and AlkL.

10. A method of producing at least one acyl amino acid in an aqueous medium comprising at least one lipase ($E_1$) and at least one glyceride and/or amino acid,

wherein the method comprises a step of:

- contacting at least one cell comprising a genetic mutation that increases the expression relative to a wild type cell of an amino acid-N-acyl-transferase ($E_2$) and an acyl-CoA synthetase ($E_3$) with the aqueous medium, and

wherein the lipase ($E_1$) is capable of hydrolysing the glyceride to at least one fatty acid and the cell is capable of converting the fatty acid and amino acid to the acyl amino acid, and
wherein the cell has a reduced fatty acid degradation capacity.

**11.** The method according to claim 10, wherein the lipase ($E_1$) is a lipase derived from any one of the microorganisms selected from the group consisting of *Candida cylindracea, Burkholderia cepacia, Pseudomonas fluorescens, Thermomyces lanuginosus,* and *Rhizomucor miehei.*

**12.** The method according to either claim 10 or 11, wherein

- $E_1$ comprises SEQ ID NO: 4 or a variant thereof;
- $E_2$ comprises SEQ ID NO: 1 or a variant thereof; and/or
- $E_3$ comprises SEQ ID NO: 2 or a variant thereof.

**13.** A method of producing at least one acyl amino acid, wherein the method comprises the step of contacting at least one cell according to any one of claims 1 to 10 to an aqueous medium comprising at least one glyceride and an amino acid.

**14.** The method according to any one of the claims 10 to 13, wherein the glyceride is a natural oil.

Europäisches Patentamt
European Patent Office
Office européen des brevets

# EUROPEAN SEARCH REPORT

Application Number
EP 15 16 6355

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 842 542 A1 (EVONIK INDUSTRIES AG) 4 March 2015 (2015-03-04) * the whole document * * see especially: * * page 8, paragraph 42 * * pages 22-23; examples 10, 11 * * pages 28-29; sequence 4 * * pages 30-31; sequence 6 * * pages 79-80; claims 1-14 * ----- | 1-14 | INV. C12P13/04 C12N9/10 C12N9/20 C12N9/00 |
| A | NAGAO, A. & KITO, M.: "Synthesis of O-Acyl-L-Homoserine by Lipase", JOURNAL OF THE AMERICAN OIL CHEMISTS SOCIETY, vol. 66, no. 5, May 1989 (1989-05), pages 710-713, XP008178149, * abstract * * page 710, column 1, line 27 - column 2, line 16 * * page 711, column 1, line 5 - column 2, line 25; table 1 * * page 712, column 1, line 18 - column 2, line 11; table 2 * ----- | 1-14 | TECHNICAL FIELDS SEARCHED (IPC) C12P C12N |
| A | WO 99/24598 A1 (HENKEL KOMMANDITGESELLSCHAFT AUF AKTIEN) 20 May 1999 (1999-05-20) * page 6, line 28 - page 7, line 25 * * page 8, lines 1-31 * * page 9, lines 7-18 * * page 11; examples 1, 3 * * page 12; claims 1-3, 5 * ----- -/-- | 1-14 | |

1

The present search report has been drawn up for all claims

| Place of search | Date of completion of the search | Examiner |
|---|---|---|
| Berlin | 20 November 2015 | Fuchs, Ulrike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 16 6355

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE UniProt [Online]<br><br>29 October 2014 (2014-10-29), ZHENG, Y. ET AL.: "SubName: Full=Lipase {ECO:0000313¦EMBL:ABV71396.1}; EC=3.1.1.3 {ECO:0000313¦EMBL:ACC59809.1}", XP002751134, Database accession no. A9XZZ0<br>* Thermomyces lanuginosus lipase gene LGY or MGY *<br>* abstract *<br>----- | 1-14 | |
| A | DATABASE UniProt [Online]<br><br>20 April 2015 (2015-04-20), NAKAYAMA, Y. ET AL.: "RecName: Full=Glycine N-acyltransferase-like protein 2; EC=2.3.1.13", XP002751135, Database accession no. Q8WU03;<br>* abstract *<br>----- | 1-14 | TECHNICAL FIELDS SEARCHED (IPC) |
| A | DATABASE UniProt [Online]<br><br>4 February 2015 (2015-02-04), SILBERGELD, E. ET AL.: "SubName: Full=Long-chain-fatty-acid--CoA ligase {ECO:0000313¦EMBL:EZJ51704.1}; EC=6.2.1.3 {ECO:0000313¦EMBL:EZJ51704.1}", XP002751136, Database accession no. A0A029IT03<br>* abstract *<br>----- | 1-14 | |

The present search report has been drawn up for all claims

1

| Place of search | Date of completion of the search | Examiner |
|---|---|---|
| Berlin | 20 November 2015 | Fuchs, Ulrike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 15 16 6355

This annex lists the patent family members relating to the patent documents cited in the above-mentioned European search report. The members are as contained in the European Patent Office EDP file on
The European Patent Office is in no way liable for these particulars which are merely given for the purpose of information.

20-11-2015

| Patent document cited in search report | Publication date | Patent family member(s) | Publication date |
|---|---|---|---|
| EP 2842542 A1 | 04-03-2015 | EP 2842542 A1 | 04-03-2015 |
| | | EP 2843043 A1 | 04-03-2015 |
| | | WO 2015028423 A1 | 05-03-2015 |
| WO 9924598 A1 | 20-05-1999 | DE 19749556 A1 | 12-05-1999 |
| | | WO 9924598 A1 | 20-05-1999 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 1483500 A
- US 20140051136 A
- US 5734070 A


### Non-patent literature cited in the description


- The DIG System Users Guide for Filter Hybridization. Boehringer Mannheim GmbH, 1993
- **ANTONENKOV, V. ; D. VAN VELDHOVEN, P., P. ; WAELKENS, E. ; MANNAERTS, G.P.** *J. Biol. Chem.,* 1997, vol. 272, 26023-26031
- **ARTHUR LESK.** Introduction to bioinformatics. 2008
- **F. M. AUSUBEL.** Current Protocols in Molecular Biology. John Wiley & Sons, Inc, 1995
- **BARKER HA.** *JBiol Chem.,* 1959, vol. 234 (2), 320-8
- **BARKER HA.** *Arch. Mikrobiol.,* 1967, vol. 59, 4-12
- **BLAIR AH ; BARKER HA.** *J. Biol. Chem.,* 1966, vol. 241, 400-408
- **BADENHORST CP.** *Drug Metab Dispos.,* 09 November 2011, vol. 40 (2), 346-52
- **BUCKEL W ; DORN U ; SEMMLER R.** *Eur. J. Biochem.,* 1981, vol. 118, 315-321
- **CHEN HP ; MARSH ENG.** *Biochemistry,* 1997, vol. 36, 14939-14945
- **CORNISH-BOWDEN.** Fundamentals of Enzyme Kinetics. Portland Press Limited, 1995
- **DUFF SM.** *Arch. Biochem. Biophys,* 2012, vol. 528, 90-101
- **ESSER D.** *Extremophiles,* 2013, vol. 17, 205-216
- **FENG Y.** *Biochemistry 41,* 22 October 2002, vol. 41 (42), 12883-90
- **FUJITA, Y. ; MATSUOKA, H. ; HIROOKA, K.** *Mol. Microbiology,* 2007, vol. 66 (4), 829-839
- **HÄRTEL U.** *Archives of Microbiology,* 1993, vol. 159 (2), 174-181
- **HAWKINS AB.** *Appl Environ Microbiol.,* 14 February 2014, vol. 80 (8), 2536-45
- **JEFFERY D.** *Insect Biochemistry,* 1988, vol. 18 (4), 347-349
- **JEREMY M BERG ; JOHN L TYMOCZKO ; LUBERT STRYER.** Biochemistry. W. H. Freeman, 2002
- **KALLIRI E.** *J. Bacteriol.,* 2008, vol. 190, 3793-3798
- **KANG, Y.** *PLOS ONE,* 2010, vol. 5 (10), e13557
- **KARMEN.** *J Clin Invest.,* 1955, vol. 34 (1), 131-3
- **KATOH et al.** *Genome Information,* 2005, vol. 16 (1), 22-33
- **LIEBL W.** *International Journal of Systematic Bacteriology,* 1991, vol. 41, 255-260
- **LIU W.** *Biochemistry,* 01 March 2005, vol. 44 (8), 2982-92
- **MAVRIDES C.** *J. Biol. Chem.,* 1975, vol. 250, 4128-4133
- **MOSKOWITZ GJ.** *Biochemistry,* 1969, vol. 8 (7), 2748-55
- **MOVVA NR ; NAKAMURA K ; INOUYE M.** *J Biol Chem.,* 10 January 1980, vol. 255 (1), 27-9
- **PARTHASARATHY A.** *Biochemistry,* 05 April 2011, vol. 50 (17), 3540-50
- **SAMBROOK ; FRITSCH ; MANIATIS.** Molecular cloning -A Laboratory Manual. Cold Spring Harbour Press, 1989
- Allgemeine Mikrobiologie. Georg Thieme Verlag, 2008
- **SLETTA H ; TØDERVIK A ; HAKVÅG S ; AUNE TE ; NEDAL A ; AUNE R ; EVENSEN G ; VALLA S ; ELLINGSEN TE ; BRAUTASET T.** *Appl Environ Microbiol.,* 01 December 2006, vol. 73 (3), 906-12
- **SÖHLING B.** *EurJ Biochem.,* 1993, vol. 212 (1), 121-7
- **TAYLOR RC.** *Microbiology,* 2010, vol. 156, 1975-1982
- **THOMPSON et al.** *Nucleic Acids Research,* 1994, vol. 22, 4637-4680
- **WALUK, D.** *FASEB J.,* 2010, vol. 24, 2795-2803
- **WANG CC.** *J. Biol. Chem.,* 1969, vol. 244, 2516-2526
- **WIESENBORN DP.** *Appl. Environ. Microbiol.,* 1988, vol. 54, 2717-2722
- **YAMASHITA H.** *Biochim. Biophys. Acta,* 2006, vol. 1761, 17-23

- **YU K.** *Enzyme Microb Technol.,* 12 June 2011, vol. 49 (3), 272-6